# EUROPÄISCHE PATENTSCHRIFT

(11) **EP 2 185 215 B1**
(45) Veröffentlichungstag und Bekanntmachung des Hinweises auf die Patenterteilung: **07.09.2016**
(21) Anmeldenummer: 08749479.5
(22) Anmeldetag: 16.05.2008
(51) Int. Cl.: A61M 1/14, A61M 1/28

(54) **VORRICHTUNG UND VERFAHREN ZUR BEHANDLUNG EINER MEDIZINISCHEN FLÜSSIGKEIT UND MEDIZINISCHE KASSETTE**
DEVICE AND METHOD FOR TREATING A MEDICAL FLUID AND MEDICAL CASSETTE
DISPOSITIF ET PROCÉDÉ DESTINÉS AU TRAITEMENT D'UN FLUIDE MÉDICAL, ET CASSETTE MÉDICALE CORRESPONDANTE

(30) Priorität: 10.09.2007 DE 102007042964
(43) Veröffentlichungstag der Anmeldung: 19.05.2010
(60) Teilanmeldung: 16179049.8
(73) Patentinhaber: Fresenius Medical Care Deutschland GmbH, 61352 Bad Homburg (DE)
(72) Erfinder: GÜNTHER, Götz, 61440 Oberursel (DE); HÄCKER, Jürgen, 61267 Neu-Anspach (DE); KÖHLER, Markus, 61440 Oberursel (DE); LAUER, Martin, 66606 St. Wendel (DE); MÜLLER, Ralf, 61348 Bad Homburg (DE); SCHNEIDER, Hans-Peter, 61267 Neu-Anspach (DE); WEBER, Tobias, 66606 St. Wendel (DE); WEIS, Manfred, 66606 St. Wendel (DE)
(74) Vertreter: Laufhütte, Dieter
(86) Internationale Anmeldenummer: PCT/EP2008/003978
(87) Internationale Veröffentlichungsnummer: WO 2009/033511

(56) Entgegenhaltungen:
- EP-A- 0 980 686
- WO-A-03/099355
- WO-A-2009/006500
- DE-A1- 10 224 750
- DE-C1- 10 157 924
- US-A1- 2005 126 998
- US-A1- 2007 112 297

## Beschreibung

Die vorliegende Erfindung betrifft eine Vorrichtung zur Behandlung einer medizinischen Flüssigkeit, welche eine Behandlungsmaschine mit einer Ankoppelfläche umfasst, wobei eine Kassette aus einem Hartteil mit flüssigkeitsführenden Kanälen, welche von einer flexiblen Folie abgedeckt sind, an die Ankoppelfläche der Behandlungsmaschine ankoppelbar ist. Weiterhin betrifft die vorliegende Erfindung eine medizinische Kassette aus einem Hartteil mit flüssigkeitsführenden Kanälen, welche von einer flexiblen Folie abgedeckt sind, wobei die Kassette an einer Ankoppelfläche einer Behandlungsmaschine ankoppelbar ist. Weiterhin betrifft die vorliegende Erfindung ein Verfahren zur Ankopplung einer Kassette aus einem Hartteil mit flüssigkeitsführenden Kanälen, welche von einer flexiblen Folie abgedeckt sind, an die Ankoppelfläche einer Behandlungsmaschine zur Behandlung einer medizinschen Flüssigkeit sowie ein Verfahren zur Überprüfung der Dichtigkeit einer solchen medizinischen Kassette.

Bei der Behandlungsmaschine kann es sich dabei um eine Blutbehandlungsmaschine handeln, wie sie z. B. bei der Hämodialyse oder bei der Peritonealdialyse zum Einsatz kommt. Die medizinische Kassette umfasst bei einem solchen Einsatz die blut- bzw. dialyseflüssigkeitsführenden Kanäle und steht über die Koppelfläche mit Aktoren und Sensoren der Behandlungsmaschine in Verbindung. Die medizinische Kassette kann so als kostengünstiges Einwegteil ausgeführt werden, während die Aktoren zur Steuerung des Flüssigkeitsstroms durch die Kassette sowie die Sensoren z. B. zur Leveldetektion oder zur Druckmessung in die Behandtungsmaschine integriert sind.

Solche als Einwegartikel ausgeführten medizinischen Kassetten bestehen dabei aus einem dünnwandigen dreidimensionalen Kunststoffhartteil mit einem ebenen umlaufenden Auflagerand und diversen Vertiefungen (Kammern, Stegen und Kanälen). In den von diesen dreidimensionalen Strukturen des Kunststoffhartteils gebildeten Kammern und Kanälen können nun medizinische Flüssigkeiten wie z. B. Dialysat oder Blut geführt werden. Die Auflageebene der Kassette wird durch eine flexible Folie, vorteilhafterweise eine Polymerfolie, die umlaufend mit dem Auflagerand des Hartteils verbunden, insbesondere verschweißt und/oder verklebt ist, flüssigkeitsdicht verschlossen. Die medizinische Kassette wird im Gebrauch mit der flexiblen Folie an die Ankoppelfläche der Behandlungsmaschine angepresst, so dass Aktoren und Sensoren der Behandlungsmaschine an der Polymerfolie aufliegen. Zusätzlich wird durch diese Anpressung die flexible Folie mit den Stegen der Kassette verpresst und sorgt so für eine fluiddichte Trennung der flüssigkeitsführenden Kanäle im Hartteil durch die Stege und die flexible Folie.

Die Ankoppelfläche der Behandlungsmaschine weist dementsprechend üblicherweise Aktoren, Sensoren und Anpresskraftübertragungsflächen auf. Die Aktoren und Sensoren der Blutbehandlungsmaschine sind dabei im angekoppelten Zustand der Kassette gegenüber den flüssigkeitsführenden Kanälen der Kassette angeordnet. Die Aktoren können hierdurch durch Herabdrücken der Folie Ventile bilden, indem die flexible Folie in Bereiche der flüssigkeitsführenden Kanäle hineingedrückt wird und diese verschließt. Die Sensoren messen z.B. Druck oder Temperatur der in den flüssigkeitsführenden Kanälen befindlichen Flüssigkeit. Die Anpresskraftübertragungsflächen pressen die flexible Folie gegen Dichtungsstege des Hartteils, welche die flüssigkeitführenden Kanäle umgeben, um diese gegeneinander und gegen den Rest der Kassette abzudichten. Dabei wird die Ankoppelfläche üblicherweise von einer planen Oberfläche eines Trägerelements, welches z.B. aus Metall gefertigt ist, in welche Aufnahmen für die Sensoren und die Aktoren vorgesehen sind, und den plan in diese Aufnahmen eingesetzte Sensoren gebildet.

Auf der Ankoppelfläche der Behandlungsmaschine ist üblicherweise eine flexible Matte angeordnet, z.B. aus Silikon oder einem anderen elastomeren Material. Dies hat den Vorteil, dass die Sensoroberflächen vor Umgebungseinflüssen geschützt werden und zudem die Maschinenoberfläche flüssigkeitsdicht und somit ideal hygienisch reinigbar ist. Die flexible Matte stellt dabei einen Teil der Behandlungsmaschine dar, an welchen die Kassette als Einwegteil angekoppelt wird. Durch die Flexibilität der Matte wird die Funktionalität der Aktoren gewährt. Zudem kann die flexible Folie über die flexible Matte gut mit der Ankoppelfläche werpresset werden, was einen guten Kontakt mit den Aktoren, Sensoren und Anpresskraftübertragungsflächen ermöglicht. Die Behandlungsmaschine kann jedoch auch ohne flexible Matte betrieben werden, so dass die flexible Folie direkt auf der Ankoppelfläche aufliegt und die Sensoren und Aktoren direkt an die Folie ankoppeln.

Bei der Ankopplung von Sensoren an die Folienoberfläche besteht dabei jedoch bei bekannten Systemen die Schwierigkeit, eine gute Kopplung zu erreichen, um korrekte Meßwerte zu erhalten. Speziell Luft, die in der Übertragungsstrecke zwischen flexibler Folie und Sensoroberfläche beim Einlegen der Kassette eingeschlossen wird, führt zu einer Verfälschung der Meßergebnisse. Dies gilt für Drucksensoren (insbesondere bei der Messung von Drücken, die kleiner sind als der Umgebungsdruck), aber auch bei der Leveldetektion und ebenfalls für Aktoren wie z. B. Ventile. Bei der Ankopplung sollten deshalb unerwünschte Lufteinschlüsse zwischen der äußeren Oberfläche der flexiblen Folie und der aufliegenden Mattenoberfläche der flexiblen Matte bzw., wenn keine Matte verwendet wird, der aufliegenden Ankoppelfläche der Behandlungsmaschine beseitigt werden. Üblicherweise erfolgt dies durch Luftabsaugung. Dabei ist jedoch die fluidische Kontaktierung dieses Raumes kompliziert. Insbesondere besteht das Problem, dass durch ein Anlegen der Folie an die Matte bzw. die Ankoppelfläche eine Selbstabdichtung der Folie stattfindet, so dass Luftinseln verbleiben.

Aus DE 101 57 924 C1 und DE. 102 24 750 A1 ist es deshalb bekannt, den Lufttransport mittels definiert vorgegebener integrierter Mattenkanäle auf der maschinenseitigen Rückseite der Maschinenmatte zu realisieren. Die Luftleitung von der Oberfläche der flexiblen Folie durch die Matte hindurch zu den maschinenseitig angeordneten Luftkanälen erfolgt dabei lokal durch durchgehende Schlitze im Bereich der Mattenkanäle. Hierdurch erfolgt der Lufttransport jedoch lediglich an genau definierten Stellen der flexiblen Folie der Kassette, an welchen die Luft durch die Schlitze in der Matte zu den maschinenseitig angeordneten Mattenkanälen abgesaugt wird. Diese Mattenkanäle müssen sich deshalb im Bereich der flüssigkeitsführenden Kanäle der Kassette befinden, um dort eine gute Absaugung zu gewährleisten, was zu Sicherheitsproblemen führen kann. Eine solche Maschinenmatte mit integrierten Mattenkanälen und Schlitzen ist zudem kostenintensiv in der Herstellung und aufwendig in der Reinigung. Auch ist so die Sensoroberfläche nicht mehr ideal vor Umgebungseinflüssen geschützt und hermetisch dicht von der Matte abgeschlossen, so dass sich auch hygienische Probleme ergeben. Zudem besteht das Bedürfnis, die Zuverlässigkeit der Luftabsaugung weiter zu verbessern, da es durch die lediglich lokale Absaugung durch die Schlitze weiterhin zum Einschluss von Luftinseln kommen kann.

Aufgabe der vorliegenden Erfindung ist es daher, eine kostengünstige, zuverlässige und hygienische Luftabsaugung zu erreichen.

Erfindungsgemäß wird diese Aufgabe von einer Vorrichtung, Kassette und Verfahren gemäß Ansprüchen 1-32 gelöst. Jegliche Beschreibung von Merkmalen als erfindungsgemäß und/oder vorteilhaft ist im Folgenden so zu verstehen, dass Schutz begehrt wird für die in den Ansprüchen definierten Gegenstände.

Diese umfaßt eine Behandlungsmaschine mit einer Ankoppelfläche, wobei eine Kassette aus seinem Hartteil mit flüssigkeitsführenden Kanälen, welche von einer flexiblen Folie abgedeckt sind, an die Ankoppelfläche der Behandlungsmaschine ankoppelbar ist. Erfindungsgemäß ist dabei im angekoppelten Zustand der Kassette zwischen der flexiblen Folie und der Ankoppelfläche zumindest in Teilbereichen eine Schicht aus einem luftdurchlässigen porösen Material angeordnet, durch welche während des Ankoppelvorgangs und/oder bei angekoppelter Kassette Luft flächig absaugbar ist. Eindungsgemäß wird damit nicht mehr wie im Stand der Technik nur punktuell durch die Schlitze in der flexiblen Matte hindurch Luft an einigen Stellen abgesaugt, so dass Luftinseln verbleiben können, sondern flächig durch die Schicht aus einem luftdurchlässigen porösen Material. Hierdurch ist eine ganzflächige Absaugung der Luft aus dem Bereich zwischen flexibler Folie und Ankoppelfläche möglich, wobei Lufteinschlüsse, welche bei lediglich lokaler Absaugung nie ausgeschlossen werden können, sicher verhindert werden. Zudem kann auf die schlecht reinigbare Ausführung der flexiblen Matte mit Mattenkanälen verzichtet werden und eine flexible Matte im wesentlichen ohne Durchbrüche oder überhaupt keine Matte verwendet werden.

Erfindungsgemäß ist es dabei möglich, dass bereits während des Ankoppelvorgangs, insbesondere kurz vor dem Abschluss des Ankoppelvorgangs, Luft besonders vorteilhaft abgesaugt werden kann. Beim Ankoppeln der Kassette wird dabei der Anpressdruck bis zu einem Maximalwert erhöht. Dabei kann bereits mit dem Absaugen begonnen werden, sobald die Kassette an der Ankoppelfläche anliegt, aber noch bevor die Kassette mit maximaler Anpresskraft angepresst wird. Bereits während dieser kurzen Phase kann ein Unterdruck gesaugt werden. Insbesondere ist das poröse Material bzw. die Profilierung in dieser Phase noch weniger stark komprimiert und damit besser luftleitend. Das Absaugen der Luft kann aber auch bei vollständig angekoppelter Kassette erfolgen.

Vorteilhafterweise ist die Luft dabei durch die Schicht aus einem luftdurchlässigen porösen Material entlang der Ebene der Schicht absaugbar. Hierdurch wird eine Luftableitung entlang der Ankoppelebene ermöglicht, während gleichzeitig die Aktoren und Sensoren senkrecht zur Ankoppelebene einwirken können. Dabei ist es ausreichend, den Raum zwischen Folie und Ankoppelfläche an einer oder mehreren Absaugstellen fluidisch zu kontaktieren und mit einer Absaugvorrichtung in Verbindung zu setzen, wobei die Luft von dort aus flächig entlang der Ankoppelebene abgesaugt wird. Soweit eine Matte vorgesehen ist, kann die flexible Matte, welche ansonsten keine Durchbrüche aufweist, an einer oder wenigen Stellen Öffnungen zum fluidischen Kontaktieren der luftdurchlässigen porösen Schicht aufweisen.

Durch die Schicht aus einem luftdurchlässigen, insbesondere porösen Material kann der Unterdruck zur Absaugung dabei im gesamten Bereich der Schicht aus einem luftdurchlässigen porösen Material wirken, was eine ganzflächige sichere Absaugung ermöglicht. Das Material der Schicht ist dabei vorteilhafterweise sowohl entlang der Hauptebene der Schicht luftdurchlässig, als auch quer zur Hauptebene der Schicht. Hierdurch kann der Lufttransport insbesondere auch in der Materialschicht entlang ihrer Hauptebene erfolgen, wodurch die Luft zwischen der Oberfläche der flexiblen Folie und der Ankoppelfläche sicher abgesaugt wird.

Vorteilhafterweise ist die Schicht aus einem luftdurchlässigen porösen Material dabei im angekoppelten Zustand der Kassette direkt auf der flexiblen Folie angeordnet. Hierdurch ergibt sich eine direkte Absaugung der Luft von der Oberfläche der flexiblen Folie, so dass eine sichere Ankopplung ermöglicht wird.

Weiterhin vorteilhafterweise umfaßt die Schicht aus einem luftdurchlässigen porösen Material dabei ein Vlies. Ein solches Vlies ermöglicht dabei den oben beschriebenen Lufttransport, wobei es für einen gleichmäßigen Kontakt zwischen Ankoppelnfläche und Folie und damit zwischen Sensoren und Aktuatoren und Folie sorgt, gleichzeitig aber die flächige Absaugung in der Ebene zwischen flexibler Folie und Ankoppelfläche bzw. flexibler Matte sicherstellt.

Weiterhin vorteilhafterweise ist die Schicht aus einem luftdurchlässigen Material dabei ganzflächig auf der flexiblen Folie angeordnet. So ergibt sich eine kostengünstige und einfache Möglichkeit, über eine ganzflächig ausgeführte Schicht aus einem luftdurchlässigen Material die Luftabsaugung zu ermöglichen. Für bestimmte Anwendungen ist es jedoch auch möglich, nur in Teilbereichen eine solche Schicht aus einem luftdurchlässigen Material vorzusehen.

Weiterhin vorteilhafterweise ist die Kassette im angekoppelten Zustand mit der Ankoppelfläche verpresst, wobei der während des Ankoppelvorgangs und/oder bei angekoppelter Kassette über die Schicht aus einem luftdurchlässigen Material orthogonal zu deren Ebene übertragene Druck die Folie fluiddicht mit den flüssigkeitsführenden Kanälen des Hartteils der Kassette verpresst, die Schicht aus einem luftdurchlässigen porösen Material entlang ihrer Ebene aber luftdurchlässig bleibt. Die Schicht aus einem luftdurchlässigen porösen Material kann so den zum Abdichten der flüssigkeitsführenden Kahäle durch das Zusammenwirken zwischen den Stegen des Hartteils und der Folie, welche auf die Stege gepresst wird, notwendigen Druck übertragen. Gleichzeitig bleibt sie jedoch entlang ihrer Ebene gasdurchlässig und sorgt so für eine flächige Absaugung der Luft zwischen Folie und Ankoppelfläche.

Weiterhin vorteilhafterweise weist die Behandlungsmaschine eine auf der Ankoppelfläche angeordnete flexible Matte, insbesondere eine Silikonmatte auf, und die Kassette ist über die flexible Matte an die Ankoppelfläche der Behandlungsmaschine ankoppelbar.

In einer vorteilhaften Ausführungsform weist die Behandlungsmaschine eine auf der Ankoppelfläche angeordnete flexible Matte, wobei die Kassette über die flexible Matte an die Ankoppelfläche der Behandlungsmaschine ankoppelbar ist und eine Schicht aus einem luftdurchlässigen Material im angekoppelten Zustand der Kassette zwischen der flexiblen Folie und der flexiblen Matte angeordnet ist. Die flexible Matte kann hierdurch die Ankoppelfläche flüssigkeitsdicht abdichten und so eine besonders einfach zu reinigende und hygienische Anordnung schaffen, während die Absaugung über die zwischen der flexiblen Matte und der flexiblen Folie angeordnete luftdurchlässige Schicht erfolgt. Als Material für die flexible Matte kann dabei Silikon oder ein anderes geeignetes Elastomer verwendet werden Als luftdurchlässige Schicht bietet sich dabei wiederum, wie bereits oben dargestellt, eine poröse Materialschicht wie z. B. ein Vlies an.

In der vorliegenden Erfindung ist es durch die flächige Absaugung der Luft entlang der Ankoppelebene jedoch auch möglich, auf die flexible Matte zwischen der flexiblen Folie der Kassette und der Ankoppelfläche der Behandlungsmaschine zu verzichten, und lediglich eine Schicht aus einem luftdurchlässigen porösen Material wie z. B. Vlies vorzusehen, so dass die flexible Folie der medizinischen Kassette über die Schicht aus einem luftdurchlässigen Material ohne eine dazwischenliegende Matte direkt auf der Ankoppelfläche der Behandlungsmaschine auflieft und die Luftabsaugung direkt zwischen Ankoppelfläche und flexibler Folie erfolgt.

Die vorliegende Erfindung umfasst weiterhin eine Vorrichtung zur Behandlung einer medizinischen Flüssigkeit, welche eine Behandlungsmaschine mit einer Ankoppelfläche und einer auf der Ankoppelfläche angeordneten flexiblen Matte umfasst, wobei eine Kassette aus einem Hartteil mit flüssigkeitsführenden Kanälen, welche von einer flexiblen Folie abgedeckt sind, über die flexible Matte an die Ankoppelfläche der Behandlungsmaschine ankoppelbar ist, wobei die flexible Matte aus einem luftdurchlässigen Material besteht und so ausgeführt ist, dass während des Ankoppelvorgangs und/oder bei angekoppelter Kassette Luft in einem Bereich der flexiblen Matte ohne Durchbrüche abgesaugt wird, und zwar entlang der Ebene der flexiblen Matte und/oder durch die flexible Matte hindurch. Durch die Verwendung eines permeablen Mattenmaterials kann so auf eine zusätzliche Schicht aus einem luftdurchlässigen Material verzichtet werden, da die flächige Absaugung über die flexible Matte selbst erfolgt. Die Zuleitung des Vakuums kann dann über entsprechende Kanäle in der Ankoppelfläche der Behandlungsmaschine erfolgen. Da dünne Silikönschichten eine gewisse Permeabilität für Luft aufweisen, kann die flexible Matte aus Silikon geformt werden und in den Bereichen, in denen Luft abgesaugt werden soll, sehr dünn ausgeführt werden, so dass durch Anlegen eines entsprechend hohen Unterdrucks Luft direkt durch die Matte hindurch abgesaugt werden kann. Auf Schlitze durch die Matte, welche das Reinigen erschweren, kann so verzichtet werden. Zudem ist die Ankoppelfläche weiterhin flüssigkeitsdicht abgedichtet.

Alternativ oder zusätzlich zur oben beschriebenen Schicht aus einem luftdurchlässigen porösen Material oder der flexiblen Matte aus einem luftdurchlässigen Material kann jedoch auch die Oberfläche der Folie bzw. die der Folie zugewandte Oberfläche der flexiblen Matte eine Profilierung aufweisen, über welche Luft abgesaugt werden kann.

Die vorliegende Erfindung umfaßt daher weiterhin eine Vorrichtung zur Behandlung einer medizinischen Flüssigkeit, welche eine Behandlungsmaschine mit einer Ankoppelfläche umfaßt, wobei eine Kassette aus einem Hartteil mit flüssigkeitsführenden Kanälen, welche von einer flexiblen Folie abgedeckt sind, an die Ankoppelfläche der Behandlungsmaschine ankoppelbar ist. Dabei weist die Oberfläche der flexiblen Folie erfindungsgemäß eine Profilierung auf, durch welche während.des Ankoppelvorgangs und/oder bei angekoppelter Kassette Luft entlang der Profilierung der Folie abgesaugt werden kann. Auch so ist ein sicheres Absaugen der Luft aus dem Bereich zwischen Folie und Ankoppelfläche bzw. Matte entlang der Ankoppelebene möglich, indem die Luft durch die durch die Profilierung der Folienoberfläche gebildeten Kanäle abgesaugt wird. Die Profilierung ermöglicht damit ebenfalls ein flächiges Absaugen der Luft und verhindert so die Bildung von Luftinseln, wobei sie zumindest in den Bereichen vorgesehen ist, wo eine luftfreie Ankopplung nötig ist. Die Profilierung in der Oberfläche der Folie ist dabei vorteilhafterweise klein genug, dass keine oder nur minimale Schwankungen in der Andruckkraft zwischen der flexiblen Folie und den Stegen des Hartteils auftreten, aber groß genug, dass die durch die Profilierung entstehenden Kanäle durch den Druck zwischen Ankoppelfläche bzw. Matte und Folie nicht gänzlich verschlossen werden, sondern luftführend bleiben.

Die Profilierung kann dabei durch Prägen der Folienoberfläche erfolgen. Alternativ kann auch direkt beim Extrudieren der Folie eine Profilierung eingebracht werden.

Weiterhin ist es möglich, eine Profilierung auf der der Folie zugewandten Seite der flexiblen Matte anzubringen. Die vorliegende Erfindung umfaßt daher weiterhin eine Vorrichtung zur Behandlung einer medizinischen Flüssigkeit, welche eine Behandlungsmaschine mit einer Ankoppelfläche und einer auf der Ankoppelfläche angeordneten flexiblen Matte, insbesondere einer Silikonmatte umfaßt, wobei eine Kassette aus einem Hartteil mit flüssigkeitsführenden Kanälen, welche von einer flexiblen Folie abgedeckt sind, über die flexible Matte an die Ankoppelfläche der Behandlungsmaschine ankoppelbar ist. Erfindungsgemäß weist die der flexiblen Folie zugewandte Oberfläche der flexiblen Matte dabei eine Profilierung auf, durch welche während des Ankoppelvorgangs und/oder bei angekoppelter Kassette Luft entlang der Profilierung der flexiblen Matte abgesaugt werden kann. Die Absaugung durch die Profilierung der Oberfläche der flexiblen Matte erfolgt dabei genauso wie die Absaugung durch die Profilierung der flexiblen Folie, wie sie oben beschrieben wurde.

Die Profilierung der Folie hat gegenüber einer Profilierung der der Folie zugewandten Oberfläche der Matte jedoch den entscheidenden Vorteil, dass die der Folie zugewandte Oberfläche der Matte glatt ausgeführt werden kann und damit leicht zu reinigen ist. Die Kassette dagegen ist ohnehin ein Einwegteil, so dass sie nach der Benutzung nicht gereinigt werden muß, sondern entsorgt wird.

Vorteilhafte Ausführungen der Profilierung, welche sowohl für die Profilierung der flexiblen Folie, als auch für die Profilierung der Mattenoberfläche anwendbar sind, werden im folgenden beschrieben:
Vorteilhafterweise weist die Profilierung eine Netzstruktur und/oder eine mäanderförmige und/oder linienförmige Struktur auf. Durch eine Netzstruktur, insbesondere vorteilhafterweise eine Wabenstruktur, kann die Luft einfach und sicher flächig abgesaugt werden. Durch eine mäanderförmige Struktur läßt sich dagegen eine gezielte Absaugung in gewissen Bereichen erreichen.

Weiterhin vorteilhafterweise ist die Profilierung anisotrop und/oder inhomogen ausgeführt. Durch die Wahl der geeigneten Struktur ist es so möglich, eine anisotrope Absaugung zu realisieren, indem z. B. die Kanäle von links nach rechts größer ausgeführt sind als von oben nach unten. Auch kann die Struktur auf der Fläche inhomogen gestaltet sein. Hierdurch ist es z. B: möglich, eine gleichmäßige Absaugung der Luft in der Fläche zu realisieren, auch wenn der Raum zwischen Folie und Matte nur an einer Stelle fluidisch mit der Absaugvorrichtung konnektiert wird.

Wichtig für die erreichbare Absaugleistung ist bei einer solchen Profilierung das Aspektverhältnis der entstehenden Kanäle. Vorteilhafterweise ist dabei die Breite der Kanäle geringer als ihre Tiefe. Durch solche schmalen, tiefen Kanäle werden beim Verpressen von Folie und Matte die Kanäle nicht verschlossen, so dass die Absaugung möglich bleibt. Je flacher die Kanäle, desto größer wäre die Gefahr der Abdichtung durch ein partielles Anliegen der Folie an der Matte. Werden die Kanäle zu breit, steigt zudem die Gefahr, dass die Verpressung auf der glatten Rückseite der Folie mit den Stegen des Hartteils zu inhomogen wird und es auf dieser Seite zu Leckagen zwischen den flüssigkeitsführenden Kanälen kommt.

In einer weiteren Ausführungsform kann die Profilierung der Folienoberfläche oder der Mattenoberfläche dabei entlang der flüssigkeitsführenden Kanäle verlaufen. Hierdurch muß nur noch ein geringeres Volumen evakuiert werden, so dass sich die für das Absaugen bzw. die für einen initialen Dichtigkeitstest benötigte Zeit reduziert. Zudem wird die Luft auch nur dort abgesaugt, wo tatsächlich eine luftfreie Konnektierung zwischen Folie und flexibler Matte nötig ist. In den Bereichen ohne flüssigkeitsführende Kanäle ist eine solche luftfreie Ankopplung dagegen nicht nötig. Die Profilierung kann sich deshalb vorteilhafterweise mäanderförmig und/oder linienförmig entlang der flüssigkeitsführenden Kanäle erstrecken.

Dabei läuft die Profilierung vorteilhafterweise jedoch in einem oder mehreren Bereichen außerhalb der flüssigkeitsführenden Kanäle zusammen, welche Absaugpunkte bilden. So kann in diesen Bereichen außerhalb der flüssigkeitsführenden Kanäle eine einfache Konnektierung z. B. mit einer Absaugvorrichtung erfolgen, während die von dem Absaugpunkt in die Bereiche der flüssigkeitsführenden Kanäle verlaufende Profilierung für eine sichere Absaugung der Luft aus diesen Bereichen sorgt.

Vorteilhafterweise verläuft die Profilierung dabei beim Übergang von dem Bereich mit flüssigkeitsführenden Kanälen zu dem Bereich außerhalb der flüssigkeitsführenden Kanäle im wesentlichen senkrecht zu der Kanalstegkante. Hierdurch wird eine gleichmäßige Verpressung der Folie mit den Kanalstegkanten gewährleistet, ohne dass die Profilierung durch die Verpressung ihre luftleitende Funktion verlieren würde.

Vorteilhafterweise weist die Profilierung in den Bereichen, in welchen sie die Kanalstegkanten nicht überquert, einen Abstand zu den Kanalstegkanten auf. So ist die Profilierung nur in den verfahrenstechnisch benötigten flüssigkeitsbenetzten Folienarealen drainagefähig. In den übrigen Bereichen ist durch den hohen Anpressdruck zwischen der Folie und dem Hartteil dagegen keine Luftabsaugung nötig.

Vorteilhafterweise ist die Profilierung weiterhin so ausgeführt, dass zwischen Bereichen der Folie mit unterschiedlichen flüssigkeitsführenden Kanälen keine direkte Verbindung besteht. Bei einer Ruptur der Folie hat dies den Vorteil, dass die Flüssigkeit sich nicht entlang der Profilierung über die gesamte Kassette ausbreiten kann. Insbesondere bleiben so auch Rupturen in einem Bereich ohne flüssigkeitsführende Kanäle folgenlos. Selbst bei einer Ruptur der Folie in einem Bereich der flüssigkeitsführenden Kanäle wird die Flüssigkeit entlang der Profilierung lediglich zum Absaugpunkt angesogen, während eine Leckage über die Kanalstegkanten hinweg zwischen den flüssigkeitsführenden Kanälen verhindert wird.

Allerdings kann eine gute und sichere Verpressung und Luftabsaugung auch bei Verwendung einer Wabenstruktur erreicht werden, welche naturgemäß nicht entlang der flüssigkeitsführenden Kanäle verläuft und bei welcher die Profilierung nicht immer senkrecht auf den Kanalstegkanten steht. Vorteil einer solchen Ausführung ist dabei die einfache und kostengünstige Herstellung.

Vorteilhafterweise ist die Kassette bei der erfindungsgemäßen Vorrichtung im angekoppelten Zustand mit der Ankoppelfläche verpresst, wobei die Folie fluiddicht mit den flüssigkeitsführenden Kanälen des Hartteils der Kassette verpresst wird, die Profilierung entlang ihrer Ebene aber einen Lufttransport erlaubt.

Die vorliegende Erfindung umfaßt weiterhin eine Vorrichtung zur Behandlung einer medizinischen Flüssigkeit, welche eine Behandlungsmaschine mit einer Ankoppelfläche und einer auf der Ankoppelfläche angeordneten flexiblen Matte, insbesondere einer Silikonmatte umfaßt, wobei eine Kassette aus einem Hartteil mit flüssigkeitsführenden Kanälen, welche von einer flexiblen Folie abgedeckt sind, über die flexible Matte an die Ankoppelfläche der Behandlungsmaschine ankoppelbar ist. Erfindungsgemäß ist dabei während des Ankoppelvorgangs und/oder bei angekoppelter Kassette Luft entlang einer Ebene zwischen der flexiblen Folie und der der flexiblen Folie zugewandten Oberfläche der flexiblen Matte absaugbar. Hierdurch kann darauf verzichtet werden, die flexible Matte mit den aufwendigen und schlecht zu reinigenden Mattenkanälen und Schlitzen auszustatten. Vielmehr kann die Luft sicher und einfach zwischen der Mattenoberfläche und der flexiblen Folie abgesaugt werden. Die Absaugung erfolgt dabei vorteilhafterweise über eine zumindest in Teilbereichen angeordnete Schicht aus einem luftdurchlässigen Material, oder aber über eine Profilierung der flexiblen Folie oder der Mattenoberfläche. Hierdurch ergeben sich die bereits oben beschriebenen Vorteile.

Erfindungsgemäß wird damit nicht mehr wie im Stand der Technik nur punktuell durch die Schlitze in der flexiblen Matte hindurch Luft an einigen Stellen abgesaugt, sondern flächig entlang der Ebene zwischen Folie und Matte. Hierdurch ist es ausreichend, den Raum zwischen Folie und Matte an einer oder wenigen Stellen fluidisch zu kontaktieren und mit der Absaugvorrichtung in Verbindung zu setzen, wobei die Luft von dort aus flächig entlang der Ankoppelebene zwischen Folie und Matte abgesaugt wird. Damit kann auf die kostenintensive Ausführung der flexiblen Matte mit Mattenkanälen verzichtet werden. Zudem wird eine sichere luftfreie Ankopplung zwischen Folie und Matte sichergestellt, ohne dass die Matte Schlitze aufweisen müßte, so dass wieder eine flüssigkeitsdichte und damit ideal hygienische reinigbare Maschinenoberfläche möglich wird.

Die Absaugung der Luft erfolgt dabei vorteilhafterweise über eine Struktur, welche im Bereich zwischen der flexiblen Folie und der flexiblen Matte angeordnet ist. So wird eine Luftleitung entlang der Ankoppelebene ermöglicht, während gleichzeitig die Aktoren und Sensoren senkrecht zur Ankoppelebene einwirken können. Insbesondere handelt es sich dabei vorteilhafterweise um eine flächige Struktur, wie z. B. die bereits oben genannte Schicht aus einem luftdurchlässigen Material bzw. eine Profilierung der Oberfläche der Folie oder der Matte. Hierdurch ist eine ganzflächige Absaugung der Luft aus diesem Bereich möglich, wobei Lufteinschlüsse, welche bei lediglich lokaler Absaugung nie ausgeschlossen werden können, sicher verhindert werden.

Weiterhin vorteilhafterweise weist die erfindungsgemäße Vorrichtung mindestens eine Absaugvorrichtung auf. Über diese Absaugvorrichtung kann ein Vakuum zur Verfügung gestellt werden, welches mit dem Bereich zwischen flexibler Folie und flexibler Matte bzw. Ankoppelfläche verbunden wird und so die Absaugung ermöglicht. Dabei kann es von Vorteil sein, die Absaugvorrichtung mehrfach auszuführen, um die Testbarkeit der Absaugeinrichtung zu verbessern.

Weiterhin vorteilhafterweise wird bei der erfindungsgemäßen Vorrichtung, unabhängig davon, über welche Struktur die flächige Absaugung realisiert wird, die luftführende Schicht an einer oder mehreren Stellen außerhalb des Bereichs der flüssigkeitsführenden Kanäle mit der Absaugvorrichtung fluidisch konnektiert. Da die Absaugung entlang der Ankoppelebene erfolgt, ist es in der vorliegenden Erfindung nicht mehr nötig, direkt im Bereich der flüssigkeitsführenden Kanäle eine fluidische Konnektierung mit einer Vakuumvorrichtung zur Verfügung zu stellen.

Weiterhin vorteilhafterweise erfolgt bei der erfindungsgemäßen Vorrichtung dabei die Absaugung über mindestens ein in der Ankoppelfläche der Behandlungsmaschine angeordnetes Ventil. Dieses wird z. B. durch Anpressen der Kassette auf die Ankoppelfläche der Behandlungsmaschine mittels eines Stößels geöffnet und saugt dann automatisch die Luft aus der luftführenden Schicht ab.

Alternativ kann die Absaugung auch durch die Kassette hindurch erfolgen. Dabei kann auf der Kassette eine Absaugöffnung mit einer Hydrophobmembran vorgesehen sein. Vorteilhafterweise erfolgt die Absaugung daher über eine oder mehrere im Hartteil der Kassette angeordnete Absaugöffnungen, welche vorteilhafterweise mit einer Hydrophobmembran ausgestattet sind. Hierdurch ist eine einfache fluidische Kontaktierung der luftführenden Schicht möglich.

Weiterhin vorteilhafterweise weist die erfindungsgemäße Vorrichtung einen optischen Sensor zur Erkennung von Leckagen insbesondere durch Streulicht-Benetzungsdetektion auf. Hierdurch ergibt sich eine vereinfachte berührungslos Leckagedetektion, insbesondere in Verbindung mit einer Profilierung der Folienoberfläche, welche durch Austreten von Flüssigkeit ihre Reflexionseigenschaften verändert, was von dem optischen Sensor erfasst werden kann.

Weiterhin vorteilhafterweise weist die erfindungsgemäße Vorrichtung eine Steuerung auf, welche eine automatische Absaugung der Luft durchführt. Die Steuerung steuert dabei die Absaugvorrichtung und sorgt so automatisch für eine luftfreie Ankopplung der medizinischen Kassette.

Weiterhin vorteilhafterweise führt die Steuerung eine automatische Überprüfung der Dichtigkeit der medizinischen Kassette durch. Dies kann, wie noch weiter unten beschrieben werden wird, durch eine Überprüfung des Unterdrucks bei der Luftabsaugung erfolgen.

Weiterhin vorteilhafterweise kommen bei den erfindungsgemäßen Vorrichtungen, wie sie bereits beschrieben wurden, eine medizinische Kassette zum Einsatz, welche im folgenden dargestellt wird.

Die Medizinische Kassette der vorliegenden Erfindung umfaßt dabei ein Hartteil mit flüssigkeitsführenden Kanälen, welche von einer flexiblen Folie abgedeckt sind, wobei die Kassette an einer Ankoppelfläche einer Behandlungsmaschine ankoppelbar ist. Dabei ist erfindungsgemäß auf der flexiblen Folie eine Struktur angeordnet, durch welche während des Ankoppelvorgangs und/oder bei angekoppelter Kassette Luft entlang der Ebene der Folienoberfläche abgesaugt werden kann. Hierdurch ist es, wie bereits ausführlich bezüglich der Vorrichtung zur Behandlung einer medizinischen Flüssigkeit dargestellt, möglich, eine sichere und zuverlässige Absaugung der Luft zwischen der flexiblen Folie und der Matte bzw. Der Ankoppelfläche der Behandlungsmaschine sicher zu stellen. Dabei ist es von besonderem Vorteil, dass die Struktur ein Bestandteil der medizinischen Kassette bildet, da diese ein Einwegteil darstellt und deshalb nach Verwendung nicht gereinigt werden muß. Damit muß auch die Struktur zur Luftabsaugung keine besonderen Bedingungen bezüglich der Reinigbarkeit erfüllen.

Vorteilhafterweise ist dabei auf der flexiblen Folie zumindest in Teilbereichen eine Schicht aus einem luftdurchlässigem, insbesondere porösen Material angeordnet, durch welche im angekoppelten Zustand der Kassette Luft flächig entlang der Ebene der Materialschicht absaugbar ist. Die Vorteile einer solchen Anordnung wurden bereits oben bezüglich der Vorrichtung dargestellt. Die Schicht aus einem luftdurchlässigen, insbesondere porösen Material, welche auf der flexiblen Folie angeordnet ist, erlaubt dabei eine sichere und gleichmäßige flächige Absaugung der Luft.

Vorteilhafterweise ist die Schicht aus einem luftdurchlässigen Material dabei ganzflächig auf der flexiblen Folie angeordnet. So läßt sich eine günstige und einfache Anordnung realisieren, bei welcher Luft ganzflächig zwischen der flexiblen Folie und der Ankoppelfläche der Behandlungsmaschine abgesaugt werden kann.

Vorteilhafterweise ist die Schicht aus einem luftdurchlässigen Material dabei in einem umlaufenden Randbereich mit der Kassette verschweißt. Hierdurch bildet die Schicht aus einem luftdurchlässigen Material eine Einheit mit der Kassette und wird sicher an dieser gehalten. Weiterhin vorteilhafterweise erfolgt die Verschweißung der Schicht aus einem luftdurchlässigen Material dabei in einem einzigen Arbeitsschritt zusammen mit der Verschweißung der Folie mit dem Hartteil So läßt sich eine kostengünstige Fertigung realisieren.

Weiterhin vorteilhafterweise bildet dabei die Verschweißung eine gasdichte Barriere entlang der Ebene der Materialschicht. Hierdurch wird durch die Verschweißung sichergestellt, dass entlang der Ebene der Materialschicht Luft nur in dem Bereich abgesaugt wird, in welchem die Kassette mit der Behandlungsmaschine verpresst ist. Ansonsten könnte Luft aus Seitenbereichen angesaugt werden und so eine Entlüftung des Bereichs zwischen der flexiblen Folie und der Behandlungsmaschine verhindern. Dabei kann ausgenutzt werden, dass beim Aufschweißen der Schicht aus einem luftdurchlässigen Material auf die Folie die Struktur der vorteilhafterweise porösen Materialschicht so verändert wird, dass eine gasdichte Barriere entsteht. Es wird also an dieser Stelle mit der flexiblen Matte oder der Ankoppelfläche gegen eine an dieser Stelle luftundurchlässige gemachte Materialschicht gedichtet. Vorteilhafterweise ist die Schicht aus einem luftdurchlässigen Material mit dem Hartteil der Kassette verschweißt. Hierzu besteht die Schicht aus einem luftdurchlässigen Material vorteilhafterweise aus einem Material, welches mit dem Kunststoff, aus welchem das Hartteil der Kassette gefertigt ist, verschweißbar ist.

Alternativ oder zusätzlich kann das Hartteil der Kassette einen umlaufenden Randbereich aufweisen, in welchem sich die Struktur, insbesondere die Schicht aus einem luftdurchlässigen Material, nicht erstreckt, so dass dieser Randbereich beim Verpressen einen Dichtsteg bildet. Dieser vom Randbereich ohne Struktur gebildete Dichtsteg stellt also sicher, dass der Raum zwischen Folie und Matte bzw. Ankoppelfläche der Behandlungsmaschine sicher entlüftet werden kann. Insbesondere kann hierzu im Randbereich der Kassette auf die Schicht aus einem luftdurchlässigen Material oder auf eine Profilierung verzichtet werden.

Weiterhin vorteilhafterweise ist dabei die flexible Folie in dem umlaufenden Randbereich mit dem Hartteil der Kassette verschweißt. Dieser umlaufende Randbereich kann damit wie im Stand der Technik einerseits zum Verschweißen mit der flexiblen Folie verwendet werden und außerdem als Dichtsteg für die Struktur dienen.

Weiterhin vorteilhafterweise ist in der vorliegenden Erfindung die Schicht aus einem luftdurchlässigen Material mit der Folie verbunden, insbesondere verklebt und/oder punktweise verschweißt und/oder kaschiert und/oder laminiert und/oder angeheftet. Hierdurch ist ein sicherer Halt der Schicht aus einem luftdurchlässigen Material auf der flexiblen Folie sichergestellt, z. B. auch dann, wenn die Schicht nicht im Randbereich mit dem Hartteil verschweißt wird.

Weiterhin vorteilhafterweise verpresst bei der medizinischen Kassette der vorliegenden Erfindung der während des Ankoppelvorgangs und/oder bei angekoppelter Kassette über die Struktur, insbesondere die Schicht aus einem luftdurchlässigen Material, orthogonal zu deren Ebene übertragene Druck die Folie fluiddicht mit den flüssigkeitsführenden Kanälen des Hartteils der Kassette. Die Struktur, insbesondere die Schicht aus einem luftdurchlässigen Material, bleibt dagegen entlang ihrer Ebene gasdurchlässig. So ist eine sichere Verpressung von Folie und Hartteil möglich, bei welcher keine Leckagen zwischen den flüssigkeitsführenden Kanälen auftreten, wobei jedoch dennoch eine sichere Luftabsaugung über die flächige Struktur möglich bleibt. Erreicht wird dies durch eine entsprechende Auslegung der Materialschicht z. B. eines Vlieses, oder aber durch die entsprechende Auslegung der Profilierung der Oberfläche der flexiblen Folie.

Weiterhin vorteilhafterweise umfaßt die Schicht aus einem luftdurchlässigen Material ein Vlies. Ein solches Vlies ist optimal zur gleichmäßigen Übertragung von Druck und zur Absaugung entlang seiner Ebene geeignet.

In einem weiteren Ausführungsbeispiel kann die Oberfläche der flexiblen Folie der medizinischen Kassette der vorliegenden Erfindung vorteilhafterweise eine Profilierung aufweisen, durch welche im angekoppelten Zustand der Kassette Luft entlang der Ebene der Folie abgesaugt werden kann. Hierdurch kann auf einfache Art und Weise erreicht werden, dass die Luft aus dem Bereich zwischen Folie und Matte bzw. zwischen Folie und Ankoppelfläche der Behandlungsmaschine sicher entfernt werden kann, ohne dass sich Lufteinschlüsse oder Luftinseln bilden. Die Anordnung der Profilierung auf der Oberfläche der Folie ist dabei von besonderem Vorteil, da die Kassette nach der Verwendung ohnehin entsorgt wird und damit nicht gereinigt werden muß.

Wie bereits bezüglich der Vorrichtung zur Behandlung einer medizinischen Flüssigkeit dargestellt, weist die Profilierung dabei vorteilhafterweise eine Netzstruktur und/oder ein mäanderförmige und/oder eine linienförmige Struktur auf. Weiterhin vorteilhafterweise ist die Profilierung anisotrop und/oder inhomogen ausgeführt. Ebenso ist es weiterhin von Vorteil, wenn die Breite der durch die Profilierung gebildeten Kanäle geringer ist als die Tiefe dieser Kanäle, da diese sich beim Verpressen mit der Behandlungsmaschine hierdurch nicht schließen und dennoch eine gleichmäßige Anpressung der glatten Rückseite der Folie mit den Stegen des Hartteils ermöglichen.

In einem weiteren vorteilhaften Ausführungsbeispiel verläuft die Profilierung dabei entlang der flüssigkeitsführenden Kanäle, wobei insbesondere auf eine mäanderförmige und/oder eine linienförmige Struktur zurückgegriffen wird. Hierdurch kann das Volumen, welches evakuiert werden muss, verringert werden.

Weiterhin vorteilhafterweise läuft dabei die Profilierung in einem oder mehreren Bereichen außerhalb der flüssigkeitsführenden Kanäle zusammen, welche Absaugpunkte bilden. Hierdurch kann in diesem Bereich eine einfache Absaugung erfolgen, während über die Profilierung entlang der flüssigkeitsführenden Kanäle eine luftfreie Ankopplung in diesem Bereich sichergestellt wird.

Vorteilhafterweise verläuft die Profilierung dabei beim Übergang von dem Bereich mit flüssigkeitsführenden Kanälen zum dem Bereich außerhalb der flüssigkeitsführenden Kanäle im wesentlichen senkrecht zu der Kanalstegkante. Hierdurch wird eine gleichmäßige Verpressung der Folie mit der Kanalstegkante bei gleichzeitig guter Luftabsaugung gewährleistet.

Vorteilhafterweise weist die Profilierung in den Bereichen, in welchen sie die Kanalstegkanten nicht überquert, ein Abstand zu den Kanalstegkanten auf. Hierdurch kann verhindert werden, dass sich die Flüssigkeit im Falle einer Ruptur entlang der Profilierung über die gesamte Kassette ausbreitet. Zudem wird eine bessere Verpressung der Folie mit den Kanalstegkanten erreicht.

Vorteilhafterweise ist die Profilierung weiterhin so ausgeführt, dass zwischen Bereichen der Folie mit unterschiedlichen flüssigkeitsführenden Kanälen keine direkte Verbindung besteht. Bei einer Ruptur der Folie hat dies den Vorteil, dass die Flüssigkeit sich nicht entlang der Profilierung über die gesamte Kassette ausbreiten kann. Insbesondere bleiben so auch Rupturen in einem Bereich ohne flüssigkeitsführende Kanäle folgenlos. Selbst bei einer Ruptur der Folie in einem Bereich der flüssigkeitsführenden Kanäle wird die Flüssigkeit entlang der Profilierung lediglich zum Absaugpunkt angesogen, während eine Leckage über die Kanalstegkanten hinweg zwischen den flüssigkeitsführenden Kanälen verhindert wird.

Weiterhin vorteilhafterweise weist das Hartteil der erfindungsgemäßen medizinischen Kassette Eingrenzungsstege auf, welche die Kanalranddichtstege verbinden und verpressungsdicht abgeschlossene Areale bilden. Vorteilhafterweise liegt die Absaugstelle dabei in einem solchen verpressungsdicht abgeschlossenen Areal, so dass bei einer Folienruptur die Leckageflüssigkeit nur dieses Areal erreicht und eine ein direkter Kontakt zwischen den einzelnen flüssigkeitsführenden Bereichen nicht möglich ist.

Weiterhin vorteilhafterweise weist die erfindungsgemäße medizinische Kassette mindestens eine am Hartteil der Kassette angeordnete Absaugöffnung auf. Über diese Absaugöffnung kann der Raum zwischen Folie und Matte bzw. Ankoppelfläche zur Absaugung fluidisch kontaktiert werden. Hierdurch kann die maschinenseitige flexible Matte durchgängig und damit ideal reinigbar ausgeführt werden.

Vorteilhafterweise ist dabei die Absaugöffnung außerhalb des Bereichs der flüssigkeitsführenden Kanäle der Kassette angeordnet. Hierdurch erhöht sich die Sicherheit, da ein Versagen der Verschweißung nur bei gleichzeitiger Folienruptur an einer Kanalstelle oder gleichzeitigem Versagen eines Dichtstegs eine Kontamination der Absaugvorrichtung bzw. der im Absaugkanal angeordneten Hydrophobmembran zur Folge hat.

Vorteilhafterweise ist die flexible Folie dabei um die Absaugöffnung herum mit dem Hartteil der Kassette verschweißt.

Dabei weist die Ringschweißnaht um die Absaugöffnung vorteilhafterweise eine Profilierung auf, um die Luftabsaugung nicht durch die Schweißnaht zu beeinträchtigen. Dies ist insbesondere bei einer Profilierung der Folienoberfläche wichtig und kann z.B. über eine entsprechende Strukturierung des Schweißsterripels erreicht werden.

Alternativ oder zusätzlich kann der Bereich der Schweißnaht auch gegenüber der Anpressebene der Kassette abgesenkt sein. Auch damit kann eine Beeinträchtigung der Luftabsaugung durch die Schweißnaht verhindert werden. Dies ist insbesondere auch bei der Verwendung einer Schicht aus luftdurchlässigem Material von besonderem Vorteil.

Weiterhin vorteilhafterweise ist dabei an der Absaugöffnung ein Hydrophobfilter angeordnet. Vorteithafterweise sind dabei die Absaugöffnung oder die Absaugöffnungen flüssigkeitsdicht mit einem bzw. mehreren Hydrophobfiltern verschlossen. Hydrophobfilter sind flüssigkeitsdicht und gleichzeitig gasdurchlässig.

Die vorliegende Erfindung umfaßt weiterhin Verfahren zur Ankopplung einer Kassette an eine Behandlungsmaschine, welche eine sichere luftfreie Ankopplung ermöglichen.

Die vorliegende Erfindung umfaßt dabei ein Verfahren zur Ankopplung einer Kassette aus einem Hartteil mit flüssigkeitsführenden Kanälen, welche von einer flexiblen Folie abgedeckt sind, an die Ankoppelfläche einer Behandlungsmaschine zur Behandlung einer medizinischen Flüssigkeit mit den Schritten: Ankoppeln der medizinischen Kassette an die Ankoppelfläche der Behandlungsmaschine, und Absaugen von Luft zwischen der flexiblen Folie und der Ankoppelfläche der Behandlungsmaschine während des Ankoppelvorgangs und/oder bei angekoppelter Kassette, wobei das Absaugen flächig über eine zumindest in Teilbereichen zwischen der flexiblen Folie und der Ankoppelfläche angeordnete Schicht aus einem luftdurchlässigen porösen Material erfolgt. Die Schritte des Ankoppelns und des Absaugens können dabei entweder hintereinander ausgeführt werden, oder aber zumindest teilweise gleichzeitig, indem noch während des Ankoppelvorgangs mit dem Absaugen begonnen wird.

Alternativ kann die Absaugung auch entlang einer Profilierung der Oberfläche der flexiblen Folie und/oder entlang einer Profilierung einer der flexiblen Folie zugewandten Oberfläche einer flexiblen Matte, über welchen die Kassette an die Ankoppelfläche angekoppelt wird, erfolgen. Hierdurch ergeben sich die bereits bezüglich der Vorrichtung dargestellten Vorteile, insbesondere eine sichere Absaugung der Luft aus dem Bereich zwischen der flexiblen Folie und der Ankoppelfläche der Behandlungsmaschine.

Weiterhin umfaßt ein Verfahren zur Ankopplung einer Kassette aus einem Hartteil mit flüssigkeitsführenden Kanälen, welche von einer flexiblen Folie abgedeckt sind, an die Ankoppelfläche einer Behandlungsmaschine zur Behandlung einer medizinischen Flüssigkeit, mit den Schritten: Ankoppeln der medizinischen Kassette an die Ankoppelfläche der Behandlungsmaschine über eine auf der Ankoppelfläche angeordnete flexible Matte, Absaugen von Luft zwischen der flexiblen Folie und der flexiblen Matte während des Ankoppelvorgangs und/oder bei angekoppelter Kassette, wobei das Absaugen in Bereichen der flexiblen Matte ohne Durchbrüche erfolgt, und zwar entlang der Ebene der flexiblen Matte und/oder durch die flexible Matte hindurch, wozu die Matte und aus einem luftdurchlässigen Material besteht.

Weiterhin umfaßt die vorliegende Erfindung ein Verfahren zur Ankopplung einer Kassette aus einem Hartteil mit flüssigkeitsführenden Kanälen, welche von einer flexiblen Folie abgedeckt sind, an die Ankoppelfläche einer Behandlungsmaschine zur Behandlung einer medizinischen Flüssigkeit, mit den Schritten: Ankoppeln der medizinischen Kassette an die Ankoppelfläche der Behandlungsmaschine über eine auf der Ankoppelfläche angeordnete flexible Matte, insbesondere Silikonmatte, und Absaugen von Luft zwischen der flexiblen Folie und der flexiblen Matte während des Ankoppelvorgangs und/oder bei angekoppelter Kassette, wobei das Absaugen entlang der Ebene zwischen der flexiblen Folie und der der flexiblen Folie zugewandten Oberfläche der flexiblen Matte erfolgt. Auch hierdurch ergeben sich die bereits oben beschriebenen Vorteile. Insbesondere kann die flexible Matte dabei günstig und einfach zu reinigend ausgeführt werden.

Vorteilhafterweise kommt dabei bei dem erfindungsgemäßen Verfahren eine medizinische Kassette und/oder eine Vorrichtung zur Behandlung einer medizinischen Flüssigkeit zum Einsatz, wie sie bereits oben dargestellt wurde.

Die vorliegende Erfindung umfaßt weiterhin ein Verfahren zur Überprüfung der Dichtigkeit einer medizinischen Kassette aus einem Hartteil mit flüssigkeitsführenden Kanälen, welche von einer flexiblen Folie abgedeckt sind, insbesondere vor dem Befüllen der medizinischen Kassette, mit den Schritten: Ankoppeln einer medizinischen Kassette an einer Ankoppelfläche einer Behandlungsmaschine, Absaugen von Luft zwischen der flexiblen Folie und der Ankoppelfläche der Behandlungsmaschine während des Ankoppelvorgangs und/oder bei angekoppelter Kassette, insbesondere flächiges Absaugen, Überprüfen der Dichtigkeit der medizinischen Kassette anhand des entstandenen Unterdrucks, wobei die Überprüfung der Dichtigkeit während und/oder nach dem Ankoppeln der Kassette erfolgt. Da sich im Falle eines Folienlecks beim Absaugen der Luft kein Vakuum aufbauen kann, ist es erfindungsgemäß möglich, eine zu hohe Leckagerate über die Unterdrucküberwachung und Unterdruckauswertung der Luftabsaugung festzustellen und anzuzeigen. Somit läßt sich - vor allem initial vor dem Füllen des Disposables und vor Beginn der Behandlung - eine Leckage der Disposablefolie erkennen. Dadurch kann das fehlerhafte Disposable durch ein intaktes ersetzt werden. So wird durch die kontinuierliche Evakuierung die Möglichkeit gegeben, Undichtigkeiten der Folie festzustellen und fehlerhafte Disposables zu ersetzen.

Dies wird insbesondere durch die flächige Absaugung der vorliegenden Erfindung erleichtert. Ohne die flächige Absaugung würde es dagegen zu Selbstabdichtungen zwischen Folie und Matte kommen, welche eine Überprüfung erschweren würden. Vorteilhafterweise kommt bei einem solchen Verfahren eine medizinische Kassette und/oder eine Vorrichtung zur Behandlung einer medizinischen Flüssigkeit, wie sie oben beschrieben wurden, zum Einsatz.

Die Anmelderin behält sich vor, einzelne oder alle Aspekte der vorliegenden Anmeldung zum Gegenstand von Teilanmeldungen zu machen, und zwar insbesondrere auch solche Aspekte, welche nicht Gegenstand der Ansprüche der vorliegenden Anmeldung sind. Die Offenbarung der Erfindung umfaßt dabei insbesondere folgende Aspekte, welche auch jeweils für sich genommen und unabhängig von den bisherigen Ausführungen Gegenstand dieser Anmeldung sind:
1. Vorrichtung zur Behandlung einer medizinischen Flüssigkeit, welche eine Behandlungsmaschine mit einer Ankoppelfläche umfasst, wobei eine Kassette aus einem Hartteil mit flüssigkeitsführenden Kanälen, welche von einer flexiblen Folie abgedeckt sind, an die Ankoppelfläche der Behandlungsmaschine ankoppelbar ist,
   dadurch gekennzeichnet,
   dass im angekoppelten Zustand der Kassette zwischen der flexiblen Folie und der Ankoppelfläche zumindest in Teilbereichen eine Schicht aus einem luftdurchlässigen porösen Material angeordnet ist, durch welche Luft während des Ankoppelvorgangs und/oder bei angekoppelter Kassette flächig absaugbar ist.
2. Vorrichtung nach Aspekt 1, wobei die Luft durch die Schicht aus einem luftdurchlässigen porösen Material entlang der Ebene der Schicht absaugbar ist
3. Vorrichtung gemäß Aspekt 1 oder 2, wobei die Schicht aus einem luftdurchlässigen porösen Material im angekoppelten Zustand der Kassette direkt auf der flexiblen Folie angeordnet ist.
4. Vorrichtung gemäß einem der Aspekte 1 bis 3, wobei die Schicht aus einem luftdurchlässigen porösen Material ein Vlies umfasst.
5. Vorrichtung gemäß einem der Aspekte 1 bis 4, wobei die Schicht aus einem luftdurchlässigen porösen Material ganzflächig auf der flexiblen Folie angeordnet ist.
6. Vorrichtung gemäß einem der Aspekte 1 bis 5, wobei die Kassette im angekoppelten Zustand mit der Ankoppelfläche verpresst ist, wobei der wähnend des Ankoppelvorgangs und/oder bei angekoppelter Kassette über die Schicht aus einem luftdurchlässigen porösen Material orthogonal zur deren Ebene übertragene Druck die Folie fluiddicht mit den flüssigkeitsführenden Kanälen des Hartteils der Kassette verpresst, die Schicht aus einem luftdurchlässigen porösen Material entlang ihrer Ebene aber luftdurchlässig bleibt.
7. Vorrichtung nach einem der Aspekte 1 bis 6, wobei die Behandlungsmaschine eine auf der Ankoppelfläche angeordnete flexible Matte aufweist, und die Kassette über die flexible Matte an die Ankoppelfläche der Behandlungsmaschine ankoppelbar ist, und die Schicht aus einem luftdurchlässigen porösen Material im angekoppelten Zustand der Kassette zwischen der flexiblen Folie und der flexiblen Matte angeordnet ist.
8. Vorrichtung zur Behandlung einer medizinischen Flüssigkeit, welche eine Behandlungsmaschine mit einer Ankoppelfläche und einer auf der Ankoppelfläche angeordneten flexiblen Matte umfasst, wobei eine Kassette aus einem Hartteil mit flüssigkeitsführenden Kanälen, welche von einer flexiblen Folie abgedeckt sind, über die flexible Matte an die Ankoppelfläche der Behandlungsmaschine ankoppelbar ist,
   dadurch gekennzeichnet,
   dass die flexible Matte aus einem luftdurchlässigen Material besteht und so ausgeführt ist, dass während des Ankoppelvorgangs und/oder bei angekoppelter Kassette Luft in einem Bereich der flexiblen Matte ohne Durchbrüche abgesaugt wird, und zwar entlang der Ebene der flexiblen Matte und/oder durch die flexible Matte hindurch.
9. Vorrichtung zur Behandlung einer medizinischen Flüssigkeit, welche eine Behandlungsmaschine mit einer Ankoppelfläche umfasst, wobei eine Kassette aus einem Hartteil mit flüssigkeitsführenden Kanälen, welche von einer flexiblen Folie abgedeckt sind, an die Ankoppelfläche der Behandlungsmaschine
   ankoppelbar ist,
   dadurch gekennzeichnet,
   dass die Oberfläche der flexiblen Folie eine Profilierung aufweist, durch welche während des Ankoppelvorgangs und/oder bei angekoppelter Kassette Luft entlang der Profilierung der Folie abgesaugt werden kann.
10. Vorrichtung zur Behandlung einer medizinischen Flüssigkeit, welche eine Behandlungsmaschine mit einer Ankoppelfläche und einer auf der AnKöppelfläche angeordneten flexiblen Matte umfasst, wobei eine Kassette aus einem Hartteil mit flüssigkeitsführenden Kanälen, welche von einer flexiblen Folie abgedeckt sind, über die flexible Matte an die Ankoppelfläche der Behandlungsmaschine ankoppelbar .ist,
   dadurch gekennzeichnet,
   dass die der flexiblen Folie zugewandete Oberfläche der flexiblen Matte eine Profilierung aufweist, durch welche während des Ankoppelvorgangs und/oder bei angekoppelter Kassette Luft entlang der Profilierung der flexiblen Matte abgesaugt werden kann.
11. Vorrichtung nach Aspekt 9 oder 10, wobei die Profilierung eine Netzstruktur und/oder eine mäanderförmige und/oder linienförmige Struktur aufweist.
12. Vorrichtung gemäß Aspekt 9 oder 10, wobei die Profilierung anisotrope und/oder inhomogen ausgeführt ist.
13. Vorrichtung gemäß Aspekt 9 oder 10, wobei die Profilierung entlang der flüssigkeitsführehden Kanäle verläuft
14. Vorrichtung gemäß Aspekt 13, wobei, die Profilierung in einem oder mehreren Bereichen außerhalb der der flüssigkeitsführenden Kanäle zusammenläuft, welche Absaugpunkte bilden.
15. Vorrichtung gemäß einem der Aspekte 9 bis 14, wobei die Proflierung beim Übergang von dem Bereich mit flüssigkeitsführenden Kanälen zum dem Bereich außerhalb der flüssigkeitsführenden Kanäle im wesentlichen senkrecht zu der Kanalstegkante verläuft.
16. Vorrichtung gemäß einem der Aspekte 13 bis 15, wobei die Profilierung in den Bereichen, in welchen sie die Kanalstegkanten nicht überquert, ein Abstand zu den Kanalstegkanten aufweist.
17. Vorrichtung gemäß einem der Aspekte 13 bis 16, wobei die Profilierung so ausgeführt ist, dass zwischen Bereichen der Folie mit unterschiedlichen flüssigkeitsführenden Kanälen keine direkte Verbindung besteht.
18. Vorrichtung gemäß einem der Aspekte 9 bis 17, wobei die Kassette im angekoppelten Zustand mit-der Ankoppelfläche verpresst ist, wobei die Folie fluiddicht mit den flüssigkeitsführenden Kanälen des Hartteils der Kassette verpresst wird, die Profilierung entlang ihrer Ebene aber einen Lufttransport erlaubt.
19. Vorrichtung zur Behandlung einer medizinischen Flüssigkeit, welche eine Behandlungsmaschine mit einer Ankoppelfläche und einer auf der Ankoppelfläche angeordneten flexiblen Matte, insbesondere einer Silikonmatte umfasst, wobei eine Kassette aus einem Hartteil mit flüssigkeitsführenden Kanälen, welche von einer flexiblen Folie abgedeckt sind. über die flexible Matte an die Ankoppelfläche der Behandlungsmaschine ankoppelbar ist,
   dadurch gekennzeichnet,
   dass während des Ankoppelvorgangs und/oder bei angekoppelter Kassette Luft entlang einer Ebene zwischen der flexiblen Folie und der der flexiblen Folie zugewandten Oberfläche der flexiblen Matte absaugbar ist.
20. Vorrichtung gemäß einem der vorangegangenen Aspekte mit mindestens einer Absaugvorrichtung.
21. Vorrichtung gemäß Aspekt 20, wobei die luftführende Schicht an einer oder mehreren Stellen außerhalb des Bereichs der flüssigkeitsführenden Kanäle mit einer Vakuumvorrichtung in Verbindung steht.
22. Vorrichtung gemäß einem der vorangegangenen Aspekte, wobei die Absaugung über mindestens ein in der Ankoppelfläche der Behandlungsmaschine angeordnetes Ventil erfolgt.
23. Vorrichtung gemäß einem der vorangegangenen Aspekte, wobei die Absaugung über eine oder mehrere im Hartteil der Kassette angeordnete Absaugöffnungen erfolgt.
24. Vorrichtung gemäß einem der vorangegangenen Aspekte, mit einem optischen Sensor zur Erkennung von Leckagen, insbesondere durch Streulicht-Benetzungsdetektion.
25. Vorrichtung gemäß einem der vorangegangenen Aspekte, mit einer Steuerung, welche eine automatische Absaugung der Luft durchführt.
26. Vorrichtung gemäß Aspekt 25, wobei die Steuerung eine automatische Überprüfung der Dichtigkeit der medizinischen Kassette durchführt.
27. Vorrichtung gemäß einem der vorangegangenen Aspekte, mit einer medizinischen Kassette gemäß einem der folgenden Aspekte.
28. Medizinische Kassette aus einem Hartteil mit flüssigkeitsführenden Kanälen, welche von einer flexiblen Folie abgedeckt sind, wobei die Kassette an einer Ankoppelfläche einer Behandlungsmaschine ankoppelbar ist,
   dadurch gekennzeichnet,
   dass auf der flexiblen Folie eine Struktur angeordnet ist, durch welche während des Ankoppelvorgangs und/oder bei angekoppelter Kassette Luft entlang der Ebene der Folienoberfläche abgesaugt werden kann.
29. Medizinische Kassette nach Aspekt 28, wobei auf der flexiblen Folie zumindest in Teilbereichen eine Schicht aus einem luftdurchlässigen, insbesondere porösen Material angeordnet ist, durch welche im angekoppelten Zustand der Kassette Luft flächig entlang der Ebene der Materialschicht absaugbar ist.
30. Medizinische Kassette nach Aspekt 29, wobei die Schicht aus einem luftdurchlässigen Material ganzflächig auf der flexiblen Folie angeordnet ist.
31. Medizinische Kassette gemäß Aspekt 29 oder 30, wobei die Schicht aus einem luftdurchlässigen Material in einem umlaufenden Randbereich mit der Kassette verschweißt ist.
32. Medizinische Kassette gemäß Aspekt 31, wobei die Verschweißung eine gasdichte Barriere entlang der Ebene der Schicht aus einem luftdurchlässigen. Material bildet.
33. Medizinische Kassette gemäß einem der Aspekte 28 bis 32, wobei die Schicht aus einem luftdurchlässigen Material mit dem Hartteil der Kassette verschweißt ist.
34. Medizinische Kassette gemäß Aspekt 28 oder 29, wobei das Hartteil der Kassette einen umlaufenden Randbereich aufweist, in welchen sich die Struktur, insbesondere die Schicht aus einem luftdurchlässigen Material, nicht erstreckt, so dass dieser beim Verpressen einen Dichtsteg bildet.
35. Medizinische Kassette gemäß Aspekt 34, wobei die flexible Folie in dem umlaufenden Randbereich mit dem Hartteil der Kassette verschweißt ist.
36. Medizinische Kassette gemäß Aspekt 29, wobei die Schicht aus einem luftdurchlässigen Material mit der Folie verbunden, insbesondere verklebt und/oder punktweise verschweißt und/oder kaschiert und/oder laminiert und/oder angeheftet ist.
37. Medizinische Kassette gemäß Aspekt 28 oder 29, wobei der während des Ankoppelvorgangs und/oder bei angekoppelter Kassette über die Struktur, insbesondere die Schicht aus einem luftdurchlässigen Material, orthogonal zu deren Ebene übertragene Druck die Folie fluiddicht mit den flüssigkeitsführenden Kanälen des Hartteils der Kassette verpresst, die Struktur, insbesondere die Schicht aus einem luftdurchlässigen Material entlang ihrer Ebene aber gasdurchlässig bleibt.
38. Medizinische Kassette gemäß Aspekt 29, wobei die Schicht aus einem luftdurchlässigen Material ein Vlies umfasst.
39. Medizinische Kassette nach Aspekt 28, wobei die Oberfläche der flexiblen Folie eine Profilierung aufweist, durch welche im angekoppelten Zustand der Kassette Luft entlang der Ebene der Folie abgesaugt werden kann.
40. Medizinische Kassette gemäß Aspekt 39, wobei die Profilierung eine Netzstruktur und/oder eine mäanderförmige und/oder eine linienförmige Struktur aufweist.
41. Medizinische Kassette gemäß Aspekt 39 oder 40, wobei die Profilierung anisotrop und/oder inhomogen ausgeführt ist.
42. Medizinische Kassette gemäß Aspekt 39 oder 40, wobei die Pröfillerung entlang der flüssigkeitsführenden Kanäle verläuft.
43. Medizinische Kassette gemäß Aspekt 42, wobei die Profilierung in einem oder mehreren Bereichen außerhalb der flüssigkeitsführenden Kanäle zusammenläuft, welche Absaugpunkte bilden.
44. Medizinische Kassette gemäß einem der Aspekte 28 bis 43, wobei die Profilierung beim Übergang von dem Bereich mit flüssigkeitsführenden Kanälen zum dem Bereich außerhalb der flüssigkeitsführenden Kanäle im wesentlichen senkrecht zu der Kanalstegkante verläuft
45. Medizinische Kassette gemäß einem der Aspekte 42 bis 44, wobei die Profilierung in den Bereichen, in welchen sie die Kanalstegkanten nicht überquert, einen Abstand zu den Kanalstegkanten aufweist.
46. Medizinische Kassette gemäß einem der Aspekte 42 bis 45, wobei die Profilierung so ausgeführt ist, dass zwischen Bereichen der Folie mit unterschiedlichen flüssigkeitsführenden Kanälen keine direkte Verbindung besteht.
47. Medizinische Kassette gemäß einem der vorangegangenen Aspekte, wobei das Hartteil Eingrenzungsstege aufweist, welche die Kanalranddichtstege verbinden und verpressungsdicht abgeschlossene Areale bildern.
48. Medizinische Kassette gemäß einem der vorangegangenen Aspekte, mit mindestens einer im Hartteil der Kassette angeordneten Absaugöffnung.
49. Medizinische Kassette gemäß Aspekt 48, wobei die Absaugöffnung außerhalb des Bereichs der flüssigkeitsführenden Kanäle angeordnet ist.
50. Medizinische Kassette gemäß Aspekt 48, wobei die flexible Folie um die Absaugöffnung herum mit dem Hartteil der Kassette verschweißt ist.
51. Medizinische Kassette gemäß Aspekt 50, wobei die Ringschweißnaht um die Absaugöffnung eine Profilierung aufweist.
52. Medizinische Kassette gemäß Aspekt 50, wobei der Bereich der Schweißnaht gegenüber der Anpressebene der Kassette abgesenkt ist.
53. Medizinische Kassette gemäß Aspekt 48, wobei an der Absaugöffnung ein Hydrophob-Filter angeordnet ist.
54. Verfahren zur Ankopplung einer Kassette aus einem Hartteil mit flüssigkeitsführenden Kanälen, welche von einer flexiblen Folie abgedeckt sind, an die Ankoppelfläche einer Behandlungsmaschine zur Behandlung einer medizinischen Flüssigkeit, mit den Schritten,
   - Ankoppeln der medizinischen Kassette an die Ankoppelfläche der Behandlungsmaschine,
   - Absaugen von Luft zwischen der flexiblen Folie und der Ankoppelfläche der Behandlungsmaschine während des Ankoppelvorgangs und/oder bei angekoppelter Kassette,
   dadurch gekennzeichnet,
   dass das Absaugen flächig über eine zumindest in Teilbereichen zwischen der flexiblen Folie und der Ankoppelfläche angeordnete Schicht aus einem luftdurchlässigen porösen Material erfolgt.
55. Verfahren zur Ankopplung einer Kassette aus einem Hartteil mit flüssigkeitsführenden Kanälen, welche von einer flexiblen Folie abgedeckt sind, an die Ankoppelfläche einer Behandlungsmaschine zur Behandlung einer medizinischen Flüssigkeit, mit den Schritten,
   - Ankoppeln der medizinischen Kassette an die Ankoppelfläche der Behandlungsmaschine über eine auf der Ankoppelfläche angeordnete flexible Matte,
   - Absaugen von Luft zwischen der flexiblen Folie und der flexiblen Matte während des Ankoppelvorgangs und/oder bei angekoppelter Kassette,
   dadurch gekennzeichnet,
   dass das Absaugen in Bereichen der flexiblen Matte ohne Durchbrüche erfolgt, und zwar entlang der Ebene der flexiblen Matte und/oder durch die flexible Matte hindurch, wozu die Matte und aus einem luftdurchlässigen Material besteht.
56. Verfahren zur Ankopplung einer Kassette aus einem Hartteil mit flüssigkeitsführenden Kanälen, welche von einer flexiblen Folie abgedeckt sind, an die Ankoppelfläche einer Behandlungsmaschine zur Behandlung einer medizinischen Flüssigkeit, mit den Schritten,
   - Ankoppeln der medizinischen Kassette an die Ankoppelfläche der Behandlungsmaschine,
   - Absaugen von Luft zwischen der flexiblen Folie und der Ankoppelfläche der Behandlungsmaschine während des Ankoppelvorgangs und/oder bei angekoppelter Kassette,
   dadurch gekennzeichnet,
   dass das Absaugen
   entlang einer Profilierung der Oberfläche der flexiblen Folie und/oder entlang einer Profilierung einer der flexiblen Folie zugewandeten Oberfläche einer flexiblen Matte, über welche die Kassette an die Ankoppelfläche ankoppelt wird, erfolgt.
57. Verfahren zur Ankopplung einer Kassette aus einem. Hartteil mit flüssigkeitsführenden Kanälen, welche von einer flexiblen Folie abgedeckt sind, an die Ankoppelfläche einer Behandlungsmaschine zur Behandlung einer medizinschen Flüssigkeit, mit den Schritten,
   - Ankoppeln der medizinischen Kassette an die Ankoppelfläche der Behandlungsmaschine über eine auf der Ankoppelfläche angeordnete flexible Matte,
   - Absaugen von Luft zwischen der flexiblen Folie und der flexiblen Matte während des Ankoppelvorgangs und/oder bei angekoppelter Kassette,
   dadurch gekennzeichnet,
   dass das Absaugen entlang der Ebene zwischen der flexiblen Folie und der der flexiblen Folie zugewandten Oberfläche der flexiblen Matte erfolgt.
58. Verfahren nach einem der Aspekte 54 bis 57, mit einer medizinischen Kassette und/oder einer Vorrichtung zur Behandlung einer medizinischen Flüssigkeit nach einem der vorangegangenen Aspekte.
59. Verfahren zur Überprüfung der Dichtigkeit einer medizinischen Kassette aus einem Hartteil mit flüssigkeitsführenden Kanälen, welche von einer flexiblen Folie abgedeckt sind, insbesondere vor dem Befüllen der medizinischen Kassette, mit den Schritten,
   - Ankoppeln der medizinischen Kassette an einer Ankoppelfläche einer Behandlungsmaschine,
   - Absaugen von Luft zwischen der flexiblen Folie und der Ankoppelfläche der Behandlungsmaschine, insbesondere flächiges Absaugen während des Ankoppelvorgangs und/oder bei angekoppelter Kassette,
   - Überprüfen der Dichtigkeit der medizinischen Kassette anhand des entstandenen Unterdrucks,
   wobei die Überprüfung der Dichtigkeit während und/oder nach dem Ankoppeln der Kassette erfolgt.
60. Verfahren nach Aspekt 59 mit einer medizinischen Kassette und/oder einer Vorrichtung zur Behandlung einer medizinischen Flüssigkeit nach einem der vorangegangenen Aspekte.

Die vorliegende Erfindung wird nun anhand von Ausführungsbeispielen und Zeichnungen näher dargestellt. Dabei zeigen:
- Figur 1:: eine Vorrichtung zur Behandlung einer medizinischen Flüssigkeit gemäß dem Stand der Technik,
- Figur 2:: eine Vorrichtung zur Behandlung einer medizinischen Flüssigkeit gemäß einem ersten Ausführungsbeispiel der vorliegenden Erfindung,
- Figur 3:: ein Vorrichtung zur Behandlung einer medizinischen Flüssigkeit gemäß einem zweiten Ausführungsbeispiel der vorliegenden Erfindung,
- Figur 4:: eine Draufsicht sowie eine Schnittansicht einer Profilierung gemäß einem dritten Ausführungsbeispiel der vorliegenden Erfindung,
- Figur 5a:: eine Draufsicht einer medizinischen Kassette gemäß einem vierten Ausführungsbeispiel der vorliegenden Erfindung,
- Figur 5b:: Eine Schnittansicht durch eine Profilierung in einem Bereich mit Dichtstegen,
- Figur 5c:: Eine Schnittansicht durch ein Ausführungsbeispiel einer erfindungsgemäßen Absaugöffnung, und
- Figur 6:: eine Vorrichtung zur Behandlung einer medizinischen Flüssigkeit gemäß einem fünften Ausführungsbeispiel der vorliegenden Erfindung.

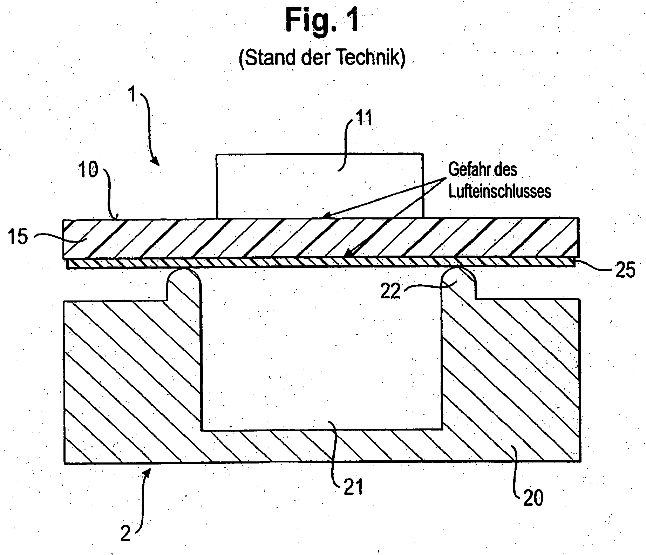

Figur 1 zeigt eine Vorrichtung zur Behandlung einer medizinischen Flüssigkeit, wie sie aus dem Stand der Technik z. B. für die Hämodialyse oder die Peritonealdialyse verwendet wird. Solche Vorrichtungen können aber auch in einer Vielzahl von anderen Einsatzgebieten verwendet werden, bei welchen eine Einwegkassette, die auch als Disposable bezeichnet wird, verwendet wird und über eine Ankoppelfläche mit Sensoren und Aktoren einer Behandlungsmaschine gekoppelt wird.

Die Behandlungsmaschine 1 weist dabei eine Ankoppelfläche 10 auf, auf welcher beispielhaft ein Sensor 11 angeordnet ist. Die Kassette 2 umfasst ein Hartteil 20 mit einem flüssigkeitsführenden Kanal 21, welcher von einer flexiblen Folie 25 abgedeckt ist, wobei der flüssigkeitsführende Kanal über seitlich angeordnete Dichtstege 22, welche mit der flexiblen Folie 25 verpresst werden, innerhalb der Kassette fluiddicht abgetrennt ist. Der Sensor 11 liegt dabei dem flüssigkeitsführenden Kanal 21 gegenüber, welcher so eine Meisskammer, im Falle eines Drucksensors eine Druckmesskammer bildet.

Zwischen der Folie 25 und der Ankoppelfläche 10 der Behandlungsmaschine 1 ist weiterhin eine flexible Silikonmatte 15 maschinenseitig angeordnet, um die Sensoroberflächen des Sensors 11 vor Umgebungseinflüssen zu schützen. Zudem ist hierdurch die Maschinenoberfläche hermetisch dicht und damit ideal hygienisch reinigbar. Alternativ kann auf die Silikonmatte 15 jedoch auch verzichtet werden, so dass die Folie 25 direkt an der Ankoppelfläche 10 der Behandlungsmaschine 1 anliegt.

Bei der Ankopplung von Sensoren an das Foliendisposable 2 besteht jedoch die Schwierigkeit, die Folie 25 an die Sensoroberfläche des Sensors 11 so anzukoppeln, dass, korrekte Meßwerte erhalten werden. Speziell Luft, die in der Übertragungsstrecke zwischen Disposablefolie 25 und Sensoroberfläche beim Einlegen der Kassette eingeschlossen wird, führt zu einer Verfälschung der Meßergebnisse. Dies gilt für Drucksensoren, aber auch z. B. bei der Leveldetektion und ebenfalls für Aktoren, wie z. B. Ventile, welche über ein Hineindrücken der flexiblen Folie 25 in die flüssigkeitsführenden Kanäle 21 des Hartteils 20 die Flüssigkeitsströme innerhalb der Kassette steuern.

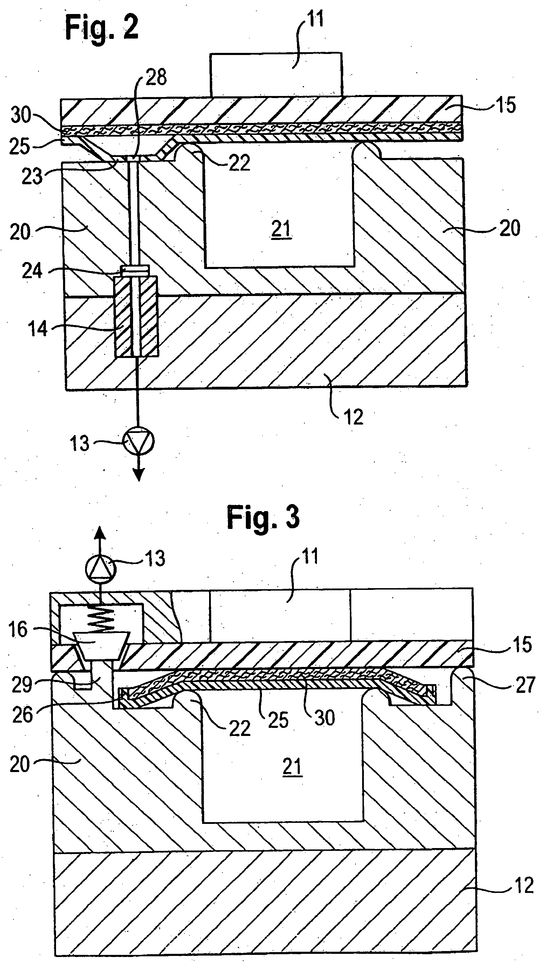

Ein erstes Ausführungsbeispiel der vorliegenden Erfindung ist nun in Figur 2 gezeigt. Dabei ermöglicht eine flächige Struktur zwischen der Folie 25 und der Matte 15 eine flächige Luftabsaugung entlang der Ankoppelebene. Hierdurch kann sichergestellt werden, dass der Raum zwischen Folie 25 und Matte 15 sicher entlüftet wird, ohne dass durch eine Selbstabdichtung durch Anlegen der Folie 25 an der Matte 15 Luftinseln eingeschlossen bleiben und die Meßergebnisse verfälschen.

In dem in Figur 2 gezeigten Ausführungsbeispiel wird die flächige Absaugung dabei dadurch erreicht, dass zwischen Folie 25 und Matte 15 eine Schicht 30 aus einem luftdurchlässigen, insbesondere porösen Material, in diesem Fall eine Vliesschicht, eingelegt wird. Eine solche Vliesschicht ist aufgrund ihrer Struktur porös, das heißt Luft kann in dieser Schicht in der Fläche strömen, auch wenn der gesamte Verbund aus Folie 25, Vlies 30 und Matte 15 z. B. durch die Dichtstege 22 stark verpresst wird.

Man erhält somit eine zwischen Folie 25 und Hartteil 20 flüssigkeitsdicht verpresste Verbindung, wobei die Schicht 30 aus einem luftdurchlässigen, insbesondere porösen Material dennoch für Luft durchlässig bleibt. Somit genügt es, den Raum zwischen Folie 25 und Matte 15 an einer einzelnen Stelle über eine Absaugöffnung 28 fluidisch mit einem Vakuumsystem 13 zu kontaktieren, um den gesamten Raum zwischen Folie 25 und Matte 15 sicher und flächig zu entlüften. Hierdurch kann eine Vielzahl von Sensoren 11 oder Aktoren sicher und luftfrei mit der Folie 25 gekoppelt werden. Die Konnektion des Vliesraums mit dem Vakuumsystem kann auf diese Weise direkt nach dem Verpressen auch für einen Foliendichtigkeitstest verwendet werden, wie er ohne eine solche Luftleitschicht nicht möglich wäre.

Die Absaugöffnung 28 ist dabei in dem ersten Ausführungsbeispiel in das Hartteil 20 der Kassette integriert und verfügt im Absaugkanal über eine Hydrophobmembran 24, welche ebenfalls in das Hartteil integriert ist. Hierdurch kann im Fehlerfall einer Folienruptur eine Kontamination der Maschine verhindert werden. Die Absaugöffnung 28 in dem Hartteil 20 der Kassette wird dabei beim Einlegen der Kassette in die Behandlungsmaschine über ein Dichtelement 14 mit in der Maschinentür 12 integrierten Absaugkanälen verbunden, welche wiederum mit einem Vakuumsystem 13 in Verbindung stehen. Die Folie weist um die Absaugöffnung 28 herum eine umlaufende Verschweißung mit dem Hartteil auf. Dabei ist der Bereich der umlaufenden Verschweißung gegenüber der Anpressebene der Kassette abgesenkt, so dass die Verschweißung der Vliesschicht 30 mit dem Hartteil keine Barriere für die Luftabsaugung darstellt.

Die fluidische Kontaktierung des Vliesraums erfolgt dabei außerhalb der flüssigkeitsführenden Gebiete der Kanalstruktur 21. Somit hat ein Versagen der Verschweißung zwischen Folie und Hartteil um die Absaugöffnung herum nur bei gleichzeitiger Folienruptur an einer Kanalstelle oder gleichzeitigen Versagen eines Dichtstegs 22 eine Kontamination der Hydrophobmembran 24 zur Folge.

In Figur 3 ist ein zweites Ausführungsbeispiel der vorliegenden Erfindung dargestellt, bei welchem die fluidische Kontaktierung nicht vom Hartteil 20 aus erfolgt, sondern von der Mattenseite aus. Hierbei ist in der Matte 15 eine Öffnung vorgesehen, welche über ein Ventil 16 mit dem Vakuumsystem 13 in Verbindung steht. Auch hier erfolgt die Kontaktierung der luftführenden Schicht 30 außerhalb des Bereichs der flüssigkeitsführenden Gebiete der Kanalstruktur 21. Das Hartteil 20 weist dabei einen Stößel 29 auf, welcher das Ventil 16 beim Einlegen der Kassette in die Behandlungsmaschine öffnet. Die Kassette weist dabei einen umlaufenden Dichtsteg 27 auf, an welchem keine Vliesschicht 30 vorgesehen ist, so dass ein nach außen geschlossener Raum zwischen Folie 25 und Matte 15 entsteht, aus welchem die Luft abgesaugt werden kann, ohne dass Leckluft von Außen einströmt.

Alternativ zu der in Figur 3 gezeigten Anordnung kann ein übermäßiges Eindringen von Leckluft in das System auch durch andere Ausführungen eines umlaufenden Dichtrandes erfolgen. Dabei kann z. B. ausgenutzt werden, dass beim Aufschweißen des Vlieses 30 auf die Folie 25 die Vliesstruktur so verändert wird, dass eine gasdichte Barriere entsteht. Es kann also an dieser Stelle ein luftundurchlässig gemachtes Vlies 30 mit der Silikonmatte 15 luftdicht verpresst werden. Idealerweise erfolgt diese Verschweißung in einem einzigen Arbeitsschritt zusammen mit der Verschweißung der Folie mit dem Hartteils 20. Dazu ist es vorteilhaft, das Vlies 30 aus einem mit dem Hartteil 20 verschweißbaren Werkstoff anzufertigen, z. B. aus PP.

Eine weitere Möglichkeit besteht darin, das Vlies kleiner als die, Folie auszuschneiden, so dass umlaufend direkt zwischen Folie und Silikonmatte gedichtet werden kann. Die Befestigung des Vlieses 30 auf der Folie kann in diesem Fall z. B. durch Kleben und/oder punktförmiges Anschweißen und/oder Kaschieren und/oder laminieren und/oder Anheften erfolgen. Zudem ist es möglich, in einem ersten Arbeitsschritt die Folie und das Vlies zu verbinden und dann in einem zweiten Schnitt die Verbindung zum Hartteil herzustellen.

Alternativ zur Verwendung des in den ersten und zweiten Ausführungsbeispielen gezeigten Vlieses kann der Lufttransport in der gewünschten Ebene erreicht werden, indem die Oberfläche der Folie 25 so profiliert wird, dass in der Folie selbst eine luftführende Schicht entsteht. Dies kann beispielsweise durch Einprägen einer Struktur in die Folie erfolgen.

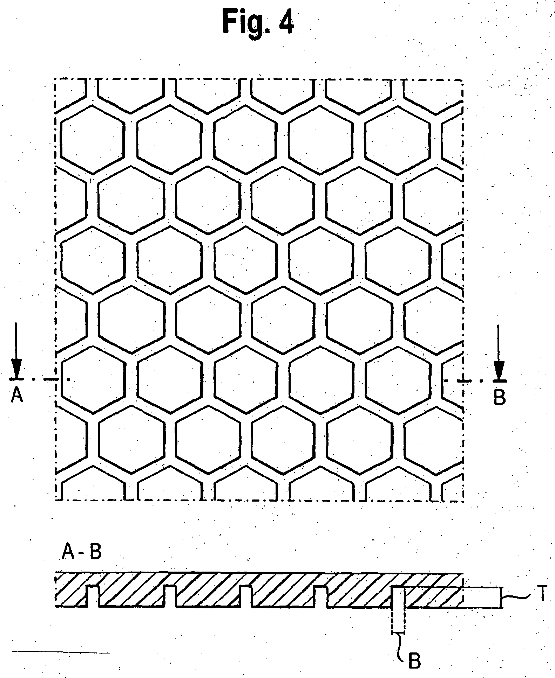

Dabei wird in einem dritten Ausführungsbeispiel eine gitterförmige Struktur in die Folie gedrückt, so dass in der Folie ein Netz von Kanälen entsteht, die durch Bereiche dickeren Materials voneinander getrennt sind. Es sind verschiedene Geometrien denkbar, wobei Figur 4 beispielhaft eine Wabenstruktur zeigt. Durch die Wahl einer geeigneten Struktur ist es auch möglich, eine anisotrope Absaugung zu realisieren, in dem z. B. die Kanäle von links nach rechts größer ausgeführt sind als von oben nach unten. Auch kann die Struktur auf der Fläche inhomogen gestaltet sein. Ebenso kann die Struktur auch mäanderförmig sein.

Wichtig für die erreichbare Absaugleistung ist hierbei die Geometrie der entstehenden Kanäle. Schmale, tiefe Kanäle werden beim Verpressen von der Silikonmatte nicht verschlossen, so dass eine Absaugung durch die Kanäle möglich bleibt. Je flacher die Kanäle sind, um so höher ist die Gefahr der Abdichtung durch ein partielles Anlegen der Folie an die Matte in den Kanälen. Werden die Kanäle zu breit, so steigt die Gefahr, dass die Verpressung auf der glatten Seite der Folie (zur Blutseite und zum Hartteil hin) zu inhomogen wird und es auf dieser Seite zu Leckagen kommt. Neben dem Prägen stehen weitere Fertigungsverfahren zur Herstellung der Profilierungen der Oberfläche der Folie zur Auswahl. So kann z. B. auch direkt beim Extrudieren der Folie eine Struktur in die Folienoberfläche eingebracht werden.

Alternativ könnte eine solche Profilierung auch in der der Folie 30 zugewandten Oberfläche der Matte 15 angeordnet werden. Auch hierdurch ließe sich ein sicheres Luftabsaugen ermöglichen. Ein Nachteil einer solchen Anordnung ist jedoch, dass die Oberfläche der Matte dann nicht mehr glatt ist und damit eine Reinigung der Mattenoberfläche erschwert wird.

Alternativ ist es ebenfalls möglich, bei Verwendung eines Vlieses 30 bzw einer Profilierung der Folienoberfläche auf die Silikonmatte 15 zu verzichten, so dass das Vlies 30 direkt zwischen Folie 25 und Ankoppelfläche 10 der Behandlungsmaschine angeordnet ist bzw. die proflierte Oberfläche der Folie 25 direkt an der Ankoppelfläche 10 der Behandlungsmaschine anliegt.

Bei der gesamtflächigen Strukturierung der Kassettenfolie bzw. durch Erzeugen einer ganzflächigen Drainageschicht zwischen Folienebene und Ankopplungsebene wird das gesamte Folienareal der Kassette bis zur umlaufenden Folienschweißnaht A bzw. bis zum äußeren umlaufenden Dichtsteg B (kassettenseitig oder maschinenseitig erzeugt) für Gase und Flüssigkeiten über alle regulären Kanalrand-Dichtstege C hinweg passierbar gemacht. Bei der anfänglichen Integritätsprüfung muß deshalb die gesamte Fläche evakuiert werden und auch die Dichtheit der umlaufenden Dichtstegs muß parallel zur eigentlich gewünschten Dichtheit der Kanalränder gewährleistet werden, damit die Behandlung freigegeben werden kann. Die initial hierzu abzusaugende Luftmenge und die dazu benötigte Zeit werden dadurch vergrößert und die Detektionssicherheit für die relevanten Folienrupturen vermindert.

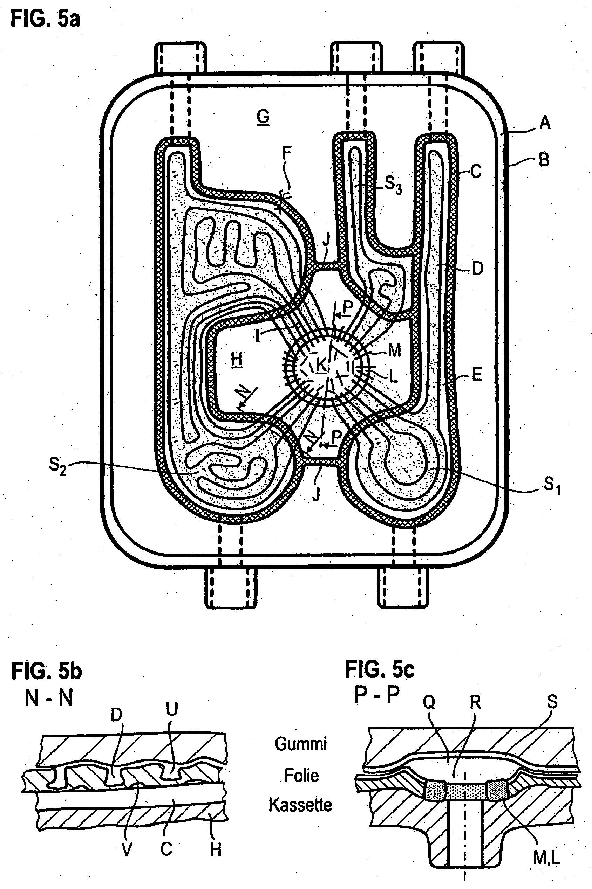

In Fig. 5a ist daher ein viertes Ausführungsbeispiel der vorliegenden Erfindung gezeigt, bei welchem eine Binnenstrukturierung der profilierten Oberfläche der Folie eingesetzt wird. Bei der Binnenstrukturierung werden nur die verfahrenstechnisch benötigten flüssigkeitsbenetzten Folienareale drainagefähig gemacht. Die Verpressung zwischen der Folienaußenseite und der Ankopplungsebene ist bedeutend geringer als die Verpressung auf den Kanalrand-Dichtstegen und in den Nicht-Flüssigkeitsarealen G und H. In diesen letzteren Arealen verfügt die Kassette über flache zur Folie parallele dichte Böden. Während die Kanalrand-Dichtstege so ausgelegt sind, dass die Flüssigkeit im Normalfall nicht übertreten kann, sind die Areale G und H so ausgelegt, dass eingedrungene Flüssigkeit keinen Raum vorfindet, da die Verpressung der Gummimatte eine annähernd vollständige Anlage der Folie sowohl zur Kassettenebene als auch zur eingesunkenen Gummi-Ebene hin bewirkt.

Da die Verpressung zwischen Folie und Gummimatte in den flüssigkeitsbenetzten Binnenarealen S1, S2 und S3 bedeutend geringer ist als die Verpressung in allen übrigen Arealen des Foliengebietes, reicht in diesem Areal eine deutlich weniger ausgeprägte Drainagestruktur aus, um sowohl die vollständige Folienankopplung als auch die vollständige Erfassung der relevanten Folienfläche im Bezug auf mögliche Rupturen zu gewährleisten. Daher kann bei der Binnenstrukturierung zu den Kanalstegkanten C ein Sicherheitsabstand E der Strukturierung D von zirka bis zu 1 mm vorgesehen werden.

Die Areale G und H werden durch das Sterilisationsverfahren mit erfaßt und hermetisch gegen die Außenwelt abgeschlossen. Tritt nun während der Behandlung, also nach einem unauffälligen initialen Integritätstest, dennoch eine Ruptur im Bereich des Areals G auf, so bleibt dies in der Regel folgenlos für die Behandlung, da die relevanten Areale S nicht betroffen sind, und da keine Möglichkeit besteht, dass Flüssigkeit in das Areal G gelangt. Tritt eine Ruptur an einem Kanaldichtsteg und über diesen hinaus auf, wie durch F skizziert, dann greifen die Funktionen der Selbstabdichtung durch die hohen Verpressungen zwischen glatter Folie und glatter Gummimatte und weiterhin die zusätzliche Verpressung am Umlaufrand. Tritt eine Ruptur in den Binnenarealen S während der Behandlung auf, so dringt das Leckagefluid in den Raum zwischen Folie und Gummimatte ein, vermindert weiter die Verpressung und wird schließlich entlang der anisotropen Strukturierung D bis an die Absaugstelle K gedrückt. Selbst das Sammel-Areal H wird dabei nur unwesentlich mit Leckageflüssigkeit aufgefüllt, da der Weg über die gezielten Leitbahnen I zur Absaugstelle K den geringsten Fließwiderstand bietet.

Die Binnenstrukturierung bietet geringere Detektionszeit und höhere Detektionsgenauigkeit beim Integritätstest vor der Befüllung mit Behandlungsflüssigkeit. Die Binnenstrukturierung bietet redundanten passiven Leckageschutz im Erstfehlerfall der Folienruptur bei Behandlung. Die Binnenstrukturierung bietet eine deutlich geringere Fläche von Folie und Gummimatte, die bei Folienruptur benetzt werden kann und zur Kontamination der Behandlungsfläche in die Behandlungsflüssigkeit und umgekehrt beitragen kann. Die Binnenstrukturierung kann die Zuverlässigkeit erhöhen und vermindert die Erfasssungszeit einer eintretenden Folienruptur bei Behandlung und erhöht damit die Sicherheit vor Kontanimation und Kreuzkontamination. Die Binnenstrukturierung kann die Wahrscheinlichkeit und das Ausmaß von möglichen Flüssigkeitsverlusten nach außen verhindern.

Weiterhin ergibt sich die Möglichkeit einer anisotropen Strukturierung: Anisotrope Folienstrukturierung oder anisotrope Gestaltung von zwischengelegten Drainageschichten bedeutet, die Intensität, die Richtung, die Funktionalität und das Nichtvorhandensein der Drainagewirküng örtlich unterschiedlich zu gestalten. Dazu gehört bereits die zuvor beschriebene Binnenstrukturierung mit unstrukturiertem Randbereich zu den Kanaldichtstegen hin.

Die nächste Möglichkeit ist die bewußte Schaffung von getrennten Strukturarealen S1...S3 mit minimierter Zahl von Überquerungen I von Kanalrand-Dichtsteg-Bereichen. I zeigt auch, wie durch die optimierte Strukturierung im Fall des Initialstests und im Falle einer Leckage ein Minimum an Volumen abgesaugt bzw. benetzt werden muß.

Die Schnittdarstellung Fig. 5b zeigt die optimierende Wirkung von Linienstrukturen, welche die Kanalrand-bichtstege senkrecht überqueren. Durch diese Anordnung kann ein Optimum an Verpressungsdichtigkeit flüssigkeitsseitig mit einem optimalen Erhalt an Drainagestrukturtiefe maschinenseitig verknüpft werden. In der Abbildung sind die zwei prinzipell negativen Ausführungen von Strukturierungen, nämlich ein teilweises Zuquetschen U der Strukturierungskanäle D und eine durch die inhomogene Kraftdurchleitung gestörte Verpressung V an den Kanalranddichtstegen C, mit extremer Übertreibung dargestellt. Parallel oder schräg die Dichtstege überquerende Strukturierungen würden stärker in den Steg und in die Gummimatte eindringen und so die Drainagewirkung und die Verpressungswirkung stärker herabsetzen.

Auch ist zu bedenken, dass es an bestimmen Stellen, beispielsweise zur optischen Messung der Flüssigkeitstrübung oder an Durchleitungsstellen für Ultraschall, erforderlich sein kann, dass die Folie glatt und/oder durchsichtig bleibt. In diesem Fall wird ein Fenster durch Weglassen von Strukturierung oder Drainageschichten geschaffen.

Weiterhin ergeben sich Möglichkeiten durch die Verwendung von Eingrenzungsstegen: Fügt man an geeigneten Stellen des Kanal- und Kammer-Layouts Verbindungsstege J zwischen den Kanalrand-Dichtstegen ein, dann erhält man neue verpressungsdicht abgeschlossene Areale H. Laufen die Drainagestrukturen in diesem Areal zusammen, dann bildet dieses Areal H zusammen mit den Flüssigkeitsarealen S1...S3 das Gesamtareal, welches auf Folienruptur vor und während der Behandlung detektiert wird, und welches für die denkbare Leckageflüssigkeit bzw. für die denkbare Kontamination und Kreuzkontamination einen passiv dichten verpressten Kanalrand als Arealgrenze aufweist. In dieses Areal H muß jede Leckage schließlich gelangen, und hier ist der bevorzugte Ort für Absaugstellen K mit Leckdetektoren und mit hydrophoben Schutzmembranen.

Eingrenzungsstege schaffen weiterhin die Möglichkeit, die Verpressungsdichtheitswirkung an den Stellen der Überquerungen I der Drainagestrukturen über die Kanaldichtränder zugunsten einer besseren Drainagewirkung etwas herabzusetzen (beispielsweise durch eine Verbreiterung des Kanalrand-Dichtstegs), ohne die Wirkung der sicheren Verpressungsdichtigkeit nach außen hin herabzusetzen.

Weiterhin schafft die vorliegende Erfindung die Möglichkeit einer nichtinvasiven Blutleckdetektion: Die Entscheidung, die Ankopplungsebene der Maschine mit einer geschlossenen Schutzschicht etwa aus Gummi zu überziehen, fordert die Konsequenz, auch einen Leckagedetektor zur frühen Entdeckung von Folienrupturen während der Behandlung als nichtinvasiv arbeitendes Gerät auszuführen. Dies ist beispielsweise durch eine dünne Gummimatte hindurch möglich über kapazitive Sensoren, über Ultraschallsensoren oder über die Detektion des bei Leckagen zusammenbrechenden Vakuums. Auf der Seite der Gegenankopplungsebene (an die Nichtfolienseite der Kassette) ist dies möglich über optische Sensoren mit Reflexanordnung. Dabei kann beispielsweise ein Farbsensor unterscheiden, ob Blut ausgetreten ist oder ob es sich um normale Feuchtigkeit handelt. Die Strukturierung der Folie kann man hier dazu ausnutzen, eine Streulicht-Benetzungsdetektion einzurichten. Bei trockener Folie wird an der Strukturierung Streulicht zurückgeworfen, welches ausbleibt, wenn die Strukturierung flüssigkeitsbenetzt ist. Bei einer solchen Anordnung auf der Nichtgummimattenseite entfällt also die Notwendigkeit einer sensorsensiblen Spezialausführung der Gummimatte.

Weiterhin ergeben sich Möglichkeiten durch Speziell strukturierte Schweißnähte und Gummimatten: Insbesondere an der Absaugstelle K entsteht durch die hierzu erforderlichen ringförmigen Schweißnähte um die Hydrophobmembran und um die Hydrophobmembran- bzw. Absauglochaussparung in der Folie das Problem, dass sowohl eine Strukturierung der Folie als auch ein Anschweißen einer darüberliegenden Drainageschicht (etwa aus Vlies) durch eine ringförmige Schweißnaht eingeebnet werden und dadurch ringförmige Sperren zwischen den Arealen H und K bilden.

Um diese Sperrmöglichkeiten aufzuheben, zeigt Fig. 5c eine Kombination aus vier möglichen Maßnahmen. Eine Delle Q in der Gummimatte setzt die Dichtverpressung gegen die Ringschweißnaht ebenso herab wie eine Delle R in der Kassettenfläche. Eine weitere ähnliche Wirkung erzielt die lokale Strukturierung S der Gummimatte im Bereich der Delle mit Rillen und Mustern, welche die Schweißnaht an mehreren Stellen drainagewirksam radial überbrücken.

Bei beiden Gummimatten-Maßnahmen ergibt sich der Nachteil einer verschlechterten Reinigbarkeit der Gummimatte. Das Pendant zu einer Strukturierung der Gummimatte im Bereich der Absaugstelle ist eine Strukturierung der Ringschweißnaht. Hierzu ist der Schweißstempel so ausgeführt, dass er ringförmig gestaltet ist, dieser Ring aber stärker konisch ausgeführt ist als die zugeordnete Anschweißflache der Kassette. Weiterhin ist die Ringfläche M mit strahlenförmig angeordneten Drainagerippen ausgerüstet, welche beim Schweißen eine gemeinsame Strukturierung von Kassetteboden und Folie hinterlassen, welche durch die Konizität der Ringflächenzuordnung nach dem Schweißen eine gezackte Ringfläche mit radial nach außen weisenden bis in die ungeschweißten Folienbereiche sich erstreckenden Linienstrukturen aufweist, welche zusammen mit den dort bereits vorhandenen strahlenförmigen Strukturen eine Gesamtstruktur bilden, welche nun nicht mehr durch die ringförmige Dichtschweißzone unterbrochen wird.

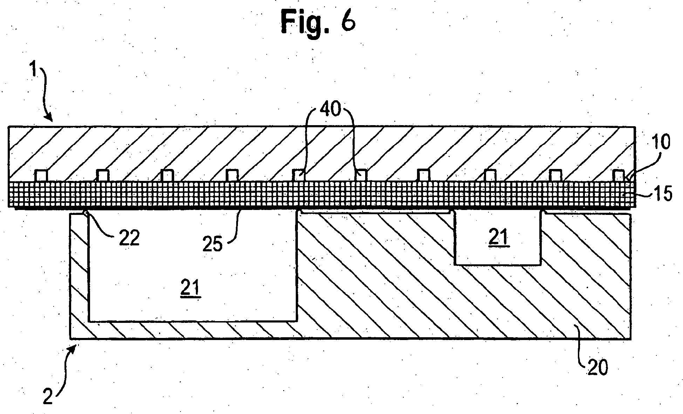

Figur 6 zeigt nun ein fünftes Ausführungsbeispiel der Vorrichtung zur Behandlung einer medizinischen Flüssigkeit der vorliegenden Erfindung, bei welcher die Absaugung nicht durch ein zusätzliches Vlies zwischen flexibler Folie 25 und flexibler Matte 15 oder eine entsprechende Profilierung der Oberflächen erfolgt, sondern durch die geeignete Wahl des Mattenmaterials über die flexible Matte 15 selbst. Die flexible Matte besteht hierfür aus einem luftdurchlässigen Material (z. B. Silikon), so dass über das an den Ansaugkanälen 40 angelegte Vakuum die Luft zwischen flexibler Matte 15 und flexibler Folie 25 direkt durch die Matte 15 selbst abgesaugt werden kann. Die großräumige Verteilung des Vakuums erfolgt dabei über die Luftkanäle 40 in der Maschinenplatte, während die permeable Matte dann eine flächige Absaugung über die gesamte Fläche ermöglicht. Auch durch diese flächige Ausführung ist ein vollständiger Verschluß der Absaugung nahezu ausgeschlossen.

Bei dieser Ausführung kann eine bereits bekannte medizinische Kassette 2 aus einem Hartteil 20 mit flüssigkeitsführenden Kanälen 21 eingesetzt werden, welche durch die flexible Folie 25 abgedichtet werden. Hierzu wird wie in bekannten Systemen die medizinische Kassette 2 mit der flexiblen Matte 15 der Behandlungmaschine verpresst wird, so dass die Dichtstege 22 für eine Abdichtung sorgen. Erfindungsgemäß ist nun aber ein flächiges Absaugen möglich, welches maschinenseitig über die Vakuumkanäle 40 sowie das permeable Mattenmaterial erfolgt

Das Mattenmaterial ist dabei vorteilhafterweise luftdurchlässig, aber flüssigkeitsdicht, um eine Verschmutzung der Ankoppelfläche zu vermeiden und die Reinigung zu erleichtern.

Durch die Möglichkeit, erfindungsgemäß kontinuierlich Luft zwischen der flexiblen Folie 25 und der Ankoppelfläche 10 abzusaugen, ergibt sich zudem die Möglichkeit, Undichtigkeiten der Folie festzustellen. Durch die flächige Evakuierung kann nach dem Verpressen der Tür im Falle eines hinreichend großen Folienlecks kein Vakuum aufgebaut werden. Es wird demnach ständig Luft aus dem noch ungefüllten Disposable in den Zwischenraum zwischen Folie und Matte bzw. Ankoppelfläche gesaugt Die zu hohe Leckagerate kann dann über die Unterdrucküberwachung und Unterdruckauswertung festgestellt und registriert werden. Die Überprüfung der Dichtigkeit kann während und/oder nach der Ankopplung an die Kassette erfolgen.

Somit lässt sich, vor allem initial vor Füllen des Disposables und vor Beginn der Behandlung, eine Leckage der Disposablefolie erkennen. Dadurch kann das fehlerhafte Disposable durch ein intaktes ersetzt werden.

Ohne die flächige Absaugung wäre eine solche Überprüfung dagegen weniger zuverlässig, da eine Selbstabdichtung zwischen Folie und Matteentstehen kann und in Bereichen mit eingeschlossenen Luftinseln weiterhin Folienlecks vorhanden sein könnten, welche über die Unterdrucküberwachung und Unterdruckauswertung nicht festgestellt würden. Im Prinzip lässt sich die erfindungsgemäße überprüfung der Dichtigkeit jedoch mit jeder Art von Evakuierung durchführen.

Zentrale Idee der vorliegenden Erfindung ist dabei das flächige Absaugen der Luft zwischen flexibler Folie und Ankoppelfläche, welche durch die unterschiedlichen Ausführungsbeispiele ermöglicht wird, indem eine luftdurchlässige Schicht, eine Pröfilierung der Oberfläche der flexiblen Folie und/oder Matte, eine Absaugung in der Ebene zwischen flexibler Matte und flexibler Folie und/oder eine Absaugung durch ein permeables Mattenmaterial vorgesehen ist.

## Patentansprüche

1. Vorrichtung zur Behandlung einer medizinischen Flüssigkeit, welche eine Behandlungsmaschine mit einer Ankoppelfläche und eine Kassette aus einem
Hartteil mit flüssigkeitsführenden Kanälen, welche von einer flexiblen Folie abgedeckt sind, umfasst, wobei die Kassette an die Ankoppelfläche der Behändlungsmaschine ankoppelbar'ist,
**dadurch gekennzeichnet,**
**dass** die der Ankoppelfläche zugewandte Oberfläche der flexiblen Folie eine Profilierung aufweist, durch welche während des Ankoppelvorgangs und/oder bei angekoppelter Kassette Luft entlang der Profilierung der Folie abgesaugt werden kann.

2. Vorrichtung zur Behandlung einer medizinischen Flüssigkeit, welche eine Behandlungsmaschine mit einer Ankoppelfläche und einer auf der Ankoppelfläche angeordneten flexiblen Matte umfasst, wobei eine Kassette aus einem Hartteil mit flüssigkeitsführenden Kanälen, welche von einer flexiblen Folie abgedeckt sind, über die flexible Matte an die Ankoppelfläche der Behandlungsmaschine ankoppelbar ist,
**dadurch gekennzeichnet,**
**dass** die flexible Matte flüssigkeitsdicht und ohne Schlitze ausgeführt ist und die der flexiblen Folie zugewandete Oberfläche der flexiblen Matte eine Profilierung aufweist, durch welche während des Ankoppelvorgangs und/oder bei angekoppelter Kassette Luft entlang der Profilierung der flexiblen Matte abgesaugt werden kann.

3. Vorrichtung nach Anspruch 1 oder 2, wobei die Profilierung eine Netzstruktur aufweist.

4. Vorrichtung gemäß einem der Ansprüche 1 bis 3, wobei die Kassette im angekoppelten Zustand mit der Ankoppelfläche verpresst ist, wobei die Folie fluiddicht mit den flüssigkeitsführenden Kanälen des Hartteils der Kassette verpresst wird, die Profilierung entlang ihrer Ebene aber einen Lufttransport erlaubt.

5. Vorrichtung gemäß einem der vorangegangenen Ansprüche mit mindestens einer Absaugvorrichtung.

6. Vorrichtung gemäß Anspruch 5, wobei die Profilierung an einer oder mehreren Stellen außerhalb des Bereichs der flüssigkeitsführenden Kanäle mit einer Vakuumvorrichtung in Verbindung steht.

7. Vorrichtung gemäß einem der vorangegangenen Ansprüche, wobei die Absaugung über mindestens ein in der Ankoppelfläche der Behandlungsmaschine angeordnetes Ventil erfolgt.

8. Vorrichtung gemäß einem der vorangegangenen Ansprüche, wobei die Absaugung über eine oder mehrere im Härtteil der Kassette angeordnete Absaugöffnungen erfolgt.

9. Vorrichtung gemäß einem der vorangegangenen Ansprüche, mit einem optischen Sensor zur Erkennung von Leckagen, insbesondere durch Streulicht-Benetzungsdetektion.

10. Vorrichtung gemäß einem der vorangegangenen Ansprüche, mit einer Steuerung, welche eine automatische Absaugung der Luft durchführt.

11. Vorrichtung gemäß Anspruch 10, wobei die Steuerung eine automatische Überprüfung der Dichtigkeit der medizinischen Kassette durchführt.

12. Medizinische Kassette aus einem Hartteil mit flüssigkeitsführenden Kanälen, welche von einer flexiblen Folie abgedeckt sind, wobei die Kassette an einer Ankoppelfläche einer Behandlungsmaschine ankoppelbar ist,
**dadurch gekennzeichnet,**
**dass** die der Ankoppelfläche zugewandte Oberfläche der flexiblen Folie eine Profilierung aufwest, durch welche im angekoppelten Zustand der Kassette Luft entlang der Ebene der Folie abgesaugt werden kann.

13. Medizinische Kassette gemäß Anspruch 12, wobei das Hartteil der Kassette einen umlaufenden Randbereich aufweist, in welchen sich die Profilierung nicht erstreckt, so dass dieser beim Verpressen einen Dichtsteg bildet.

14. Medizinische Kassette gemäß Anspruch 13, wobei die flexible Folie in dem umlaufenden Randbereich mit dem Hartteil der Kassette verschweißt ist.

15. Medizinische Kassette gemäß Anspruch 12, wobei die Profilierung eine Netzstruktur aufweist.

16. Medizinische Kassette gemäß Anspruch 12, wobei die Profilierung eine mäanderförmige und/oder eine linienförmige Struktur aufweist.

17. Medizinische Kassette gemäß Anspruch 12 oder 16, wobei die Profilierung anisotrop und/oder inhomogen ausgeführt ist.

18. Medizinische Kassette gemäß Anspruch 12 oder 16, wobei die Profilierung entlang der flüssigkeitsführenden Kanäle verläuft.

19. Medizinische Kassette gemäß Anspruch 18, wobei die Profilierung in einem oder mehreren Bereichen außerhalb der flüssigkeitsführenden Kanäle zusammenläuft, welche Absaugpunkte bilden.

20. Medizinische Kassette gemäß einem der Ansprüche 11 bis 17, wobei die Profilierung beim Übergang von dem Bereich mit flüssigkeitsführenden Kanälen zum dem Bereich außerhalb der flüssigkeitsführenden. Kanäle im wesentlichen senkrecht zu der Kanalstegkante verläuft.

21. Medizinische Kassette gemäß einem der Ansprüche 18 bis 20, wobei die Profilierung in den Bereichen, in welchen sie die Kanalstegkanten nicht überquert, einen Abstand zu den Kanalstegkanten aufweist.

22. Medizinische Kassette gemäß einem der Ansprüche 18 bis 21, wobei die Profilierung so ausgeführt ist, dass zwischen Bereichen der Folie mit unterschiedlichen flüssigkeitsführenden Kanälen keine direkte Verbindung besteht.

23. Medizinische Kassette gemäß einem der vorangegangenen Ansprüche, wobei das Hartteil Eingrenzungsstege aufweist, welche die Kanalranddichtstege verbinden und verpressungsdicht abgeschlossene Areale bilden.

24. Medizinische Kassette gemäß einem der vorangegangenen Ansprüche, mit mindestens einer im Hartteil der Kassette angeordneten Absaugöffnung.

25. Medizinische Kassette gemäß Anspruch 24, wobei die Absaugöffnung außerhalb des Bereichs der flüssigkeitsführenden Kanäle angeordnet ist.

26. Medizinische Kassette gemäß Anspruch 24, wobei die flexible Folie um die Absaugöffnung herum mit dem Hartteil der Kassette verschweißt ist.

27. Medizinische Kassette gemäß Anspruch 26, wobei die Ringschweißnaht um die Absaugöffnung eine Profilierung aufweist.

28. Medizinische Kassette gemäß Anspruch 26, wobei der Bereich der Schweißnaht gegenüber der Anpressebene der Kassette abgesenkt ist.

29. Medizinische Kassette gemäß Anspruch 24, wobei an der Absaugöffnung ein Hydrophob-Filter angeordnet ist.

30. Verfahren zur Ankopplung einer Kassette aus einem Hartteil mit fiüssigkeitsführenden Kanälen, welche von einer flexiblen Folie abgedeckt sind, an die Ankoppelfläche einer Behandlungsmaschine zur Behandlung einer medizinischen Flüssigkeit, mit den Schritten,
- Ankoppeln der medizinischen Kassette an die Ankoppelfläche der Behandlungsmaschine,
- Absaugen von Luft zwischen der flexiblen Folie und der Ankoppelfläche der Behand lungsmaschine während des Ankoppelvorgangs und/oder bei angekoppelter Kassette,
**dadurch gekennzeichnet,**
**dass** das Absaugen
entlang einer Profilierung der den Ankoppelfläche zugewandten Oberfläche der flexiblen Folie und/oder entlang einer Profilierung einer der flexiblen Folie zugewandeten Oberfläche einer flexiblen Matte, die flüssigkeitsdicht und ohne Schlitze ausgeführt ist und über welche die Kassette an die Ankoppelfläche ankoppelt wird, erfolgt.

31. Verfahren nach Anspruch 30, mit einer medizinischen Kassette und/oder einer Vorrichtung zur Behandlung einer medizinischen Flüssigkeit nach einem der vorangegangenen Ansprüche.

32. Verfahren nach Anspruch 30 oder 31 zur Überprüfung der Dichtigkeit einer medizinischen Kassette aus einem Hartteil mit flüssigkeitsführenden Kanälen, welche von einer flexiblen Folie abgedeckt sind, insbesondere vor dem Befüllen der medizinischen Kassette, mit den Schritten,
- Ankoppeln der medizinischen Kassette an einer Ankoppelfläche einer Behandlungsmaschine,
- Absaugen von Luft zwischen der flexiblen Folie und der Ankoppelfläche der Behandlungsmaschine, insbesondere flächiges Absaugen während des Ankoppelvorgangs und/oder bei angekoppelter Kassette,
- Überprüfen der Dichtigkeit der medizinischen Kassette anhand des entstandenen Unterdrucks,
wobei die Überprüfung der Dichtigkeit während und/oder nach dem Ankoppeln der Kassette erfolgt.

## Claims

1. An apparatus for treating a medical liquid which includes a treatment machine having a coupling surface, and a cassette made of a hard part having liquid-conducting passages which are covered by a flexible film, with the cassette being able to be coupled to the coupling surface of the treatment machine,
**characterized in that**,
the surface of the flexible film facing the coupling surface has a structuring through which air can be sucked out along the structuring of the film during the coupling process and/or with a coupled cassette.

2. An apparatus for treating a medical liquid which includes a treatment machine having a coupling surface and a flexible mat arranged on the coupling surface, with a cassette made of a hard part having liquid-conducting passages which are covered by a flexible film being able to be coupled to the coupling surface of the treatment machine via the flexible mat, **characterized in that**
the flexible mat is designed leaktight and without slots and the surface of the flexible mat facing the flexible film has a structuring through which air can be sucked out along the structuring of the flexible mat during the coupling process and/or with a coupled cassette.

3. An apparatus in accordance with either of claims 1 or 2, wherein the structuring has a net structure.

4. An apparatus in accordance with one of the claims 1 to 3, wherein the cassette is pressed with the coupling surface in the coupled state, with the film being pressed in a fluid-tight manner with the liquid-conducting passages of the hard part of the cassette, but with the structuring along its plane allowing air transport.

5. An apparatus in accordance with one of the preceding claims, comprising at least one suction device.

6. An apparatus in accordance with claim 5, wherein the structuring is in communication with a vacuum device at one or more points outside the region of the liquid-conducting passages.

7. An apparatus in accordance with one of the preceding claims, wherein the suction takes place via at least one valve arranged in the coupling surface of the treatment machine.

8. An apparatus in accordance with one of the preceding claims, wherein the suction takes place via one or more suction openings arranged in the hard part of the cassette.

9. An apparatus in accordance with one of the preceding claims, comprising an optical sensor for the recognition of leaks, in particular by scattered light wetting detection.

10. An apparatus in accordance with one of the preceding claims, comprising a control which carries out an automatic suction of the air.

11. An apparatus in accordance with claim 10, wherein the control carries out an automatic check of the leaktightness of the medical cassette.

12. A medical cassette made of a hard part having liquid-conducting passages which are covered by a flexible film, with the cassette being able to be coupled to the coupling surface of the treatment machine,
**characterized in that**
the surface of the flexible film facing the coupling surface has a structuring through which air can be sucked out along the plane of the film in the coupled state of the cassette.

13. A medical cassette in accordance with either of claim 12, wherein the hard part of the cassette has a peripheral marginal region into which the structuring does not extend so that this marginal region forms a sealing web on pressing.

14. A medical cassette in accordance with claim 13, wherein the flexible film is welded to the hard part of the cassette in the peripheral marginal region.

15. A medical cassette in accordance with claim 12, wherein the structuring has a net structure.

16. A medical cassette in accordance with claim 12, wherein the structuring has a meander-like structure and/or a linear structure.

17. A medical cassette in accordance with either of claims 12 or 16, wherein the structuring is made anisotropic and/or inhomogeneous.

18. A medical cassette in accordance with either of claims 12 or 16, wherein the structuring extends along the liquid-conducting passages.

19. A medical cassette in accordance with claim 18, wherein the structuring converges in one or more regions outside the liquid-conducting passages which form suction points.

20. A medical cassette in accordance with one of the claims 11 to 17, wherein the structuring extends substantially perpendicular to the passage web edge on the transition from the region with liquid-conducting passages to the region outside the liquid-conducting passages.

21. A medical cassette in accordance with one of the claims 18 to 20, wherein the structuring has a spacing from the passage web edges in the regions in which it does not traverse the passage web edges.

22. A medical cassette in accordance with one of the claims 18 to 21, wherein the structuring is made such that there is no direct connection between regions of the film with different liquid-conducting passages.

23. A medical cassette in accordance with one of the preceding claims, wherein the hard part has containment webs which connect the passage rim sealing webs and form areas sealingly pressed closed.

24. A medical cassette in accordance with one of the preceding claims, comprising at least one suction opening arranged in the hard part of the cassette.

25. A medical cassette in accordance with claim 24, wherein the suction opening is arranged outside the region of the liquid-conducting passages.

26. A medical cassette in accordance with claim 24, wherein the flexible film is welded to the hard part of the cassette around the suction opening.

27. A medical cassette in accordance with claim 26, wherein the annular weld seam has a structuring around the suction opening.

28. A medical cassette in accordance with claim 26, wherein the region of the weld seam is lowered with respect to the pressing plane of the cassette.

29. A medical cassette in accordance with claim 24, wherein a hydrophobic filter is arranged at the suction opening.

30. A method for coupling a cassette made of a hard part having liquid-conducting passages which are covered by a flexible film to the coupling surface of a treatment machine for treating a medical liquid comprising the steps:
- coupling the medical cassette to the coupling surface of the treatment machine;
- suction of air between the flexible film and the coupling surface of the treatment machine during the coupling process and/or with a coupled cassette,
**characterized in that**
the suction takes place along a structuring of the surface of the flexible film facing the coupling surface and/or along a structuring of a surface of a flexible mat facing the flexible film which is designed fluid tight and without slots and via which the cassette is coupled to the coupling surface.

31. A method in accordance with claim 30, comprising a medical cassette and/or an apparatus for treating a medical liquid in accordance with one of the preceding claims.

32. A method in accordance with claims 30 or 31 for checking the leaktightness of a medical cassette made of a hard part having liquid-conducting passages which are covered by a flexible film, in particular before the filling of the medical cassette, comprising the steps:
- coupling the medical cassette to a coupling surface of a treatment machine;
- suction of air between the flexible film and the coupling surface of the treatment machine, in particular areal suction during the coupling process and/or with a coupled cassette;
- checking the leaktightness of the medical cassette with reference to the created vacuum,
wherein the check of the leaktightness takes place during and/or after the coupling of the cassette.

## Revendications

1. Dispositif destiné au traitement d'un fluide médical, qui comprend une machine de traitement dotée d'une surface de couplage et une cassette constituée d'une partie dure avec des canaux de transfert de fluide, qui sont recouverts d'un film flexible, la cassette pouvant être couplée à la surface de couplage de la machine de traitement,
**caractérisé en ce que**
la surface du film flexible faisant face à la surface de couplage comporte un profilage, par lequel de l'air peut être aspiré le long du profilage du film pendant le procédé de couplage et/ou lorsque la cassette est couplée.

2. Dispositif destiné au traitement d'un fluide médical, qui comprend une machine de traitement dotée d'une surface de couplage et un mat flexible disposé sur la surface de couplage, une cassette constituée d'une partie dure avec des canaux de transfert de fluide, qui sont recouverts d'un film flexible, pouvant être couplée à la surface de couplage de la machine de traitement par le biais du mat flexible,
**caractérisé en ce que**
le mat flexible est réalisé étanche aux liquides et sans fente et la surface du mat flexible faisant face au film flexible comporte un profilage, par lequel de l'air peut être aspiré le long du profilage du mat flexible pendant le procédé de couplage et/ou lorsque la cassette est couplée.

3. Dispositif selon la revendication 1 ou 2, dans lequel le profilage comporte une structure de maillage.

4. Dispositif selon l'une des revendications 1 à 3, dans lequel la cassette, dans l'état couplé, est compressée avec la surface de couplage, le film étant compressé de manière étanche aux fluides avec les canaux de transfert de fluide de la partie dure de la cassette, le profilage permettant toutefois un transport de l'air le long de son niveau.

5. Dispositif selon l'une des revendications précédentes, comprenant au moins un dispositif d'aspiration.

6. Dispositif selon la revendication 5, dans lequel le profilage est en liaison avec un dispositif à vide à un ou plusieurs endroits situés hors de la zone des canaux de transfert de fluide.

7. Dispositif selon l'une des revendications précédentes, dans lequel l'aspiration se fait par le biais d'au moins une vanne disposée dans la surface de couplage de la machine de traitement.

8. Dispositif selon l'une des revendications précédentes, dans lequel l'aspiration se fait par le biais d'un ou plusieurs orifices d'aspiration disposés dans la partie dure de la cassette.

9. Dispositif selon l'une des revendications précédentes, comprenant un capteur optique destiné à détecter des fuites, en particulier par détection de lumière diffuse / mouillage.

10. Dispositif selon l'une des revendications précédentes, comprenant une commande, qui exécute une aspiration automatique de l'air.

11. Dispositif selon la revendication 10, dans lequel la commande exécute une vérification automatique de l'étanchéité de la cassette médicale.

12. Cassette médicale constituée d'une partie dure dotée de canaux de transfert de fluide, qui sont recouverts d'un film flexible, la cassette pouvant être couplée à une surface de couplage d'une machine de traitement, **caractérisée en ce que**
la surface du film flexible faisant face à la surface de couplage comporte un profilage, par lequel de l'air peut être aspiré le long du niveau du film lorsque la cassette est couplée.

13. Cassette médicale selon la revendication 12, dans laquelle la partie dure de la cassette comporte une zone de bord périphérique, dans laquelle le profilage ne s'étend pas, de telle sorte que celle-ci forme un épaulement d'étanchéité lors de la compression.

14. Cassette médicale selon la revendication 13, dans laquelle le film flexible est soudé à la partie dure de la cassette dans la zone de bord périphérique.

15. Cassette médicale selon la revendication 12, dans laquelle le profilage comporte une structure de maillage.

16. Cassette médicale selon la revendication 12, dans laquelle le profilage comporte une structure sinueuse et/ou en forme de ligne.

17. Cassette médicale selon la revendication 12 ou 16, dans laquelle le profilage est réalisé de manière anisotrope et/ou de manière non homogène.

18. Cassette médicale selon la revendication 12 ou 16, dans laquelle le profilage s'étend le long des canaux de transfert de fluide.

19. Cassette médicale selon la revendication 18, dans laquelle le profilage converge en une ou plusieurs zones hors des canaux de transfert de fluide, qui forment des points d'aspiration.

20. Cassette médicale selon l'une des revendications 11 à 17, dans laquelle le profilage s'étend sensiblement perpendiculairement au bord d'épaulement du canal au niveau de la transition de la zone avec des canaux de transfert de fluide à la zone hors des canaux de transfert de fluide.

21. Cassette médicale selon l'une des revendications 18 à 20, dans laquelle le profilage est à une distance des bords d'épaulement du canal dans les zones dans lesquelles il ne franchit pas les bords d'épaulement du canal.

22. Cassette médicale selon l'une des revendications 18 à 21, dans laquelle le profilage est réalisé de telle manière qu'aucune liaison directe n'existe entre des zones du film dotées de différents canaux de transfert de fluide.

23. Cassette médicale selon l'une des revendications précédentes, dans laquelle la partie dure comporte des épaulements de limitation, qui relient les épaulements d'étanchéité de bord de canal et forment des aires fermées de manière étanche par compression.

24. Cassette médicale selon l'une des revendications précédentes, comprenant au moins un orifice d'aspiration disposé dans la partie dure de la cassette.

25. Cassette médicale selon la revendication 24, dans laquelle l'orifice d'aspiration est disposé hors de la zone des canaux de transfert de fluide.

26. Cassette médicale selon la revendication 24, dans laquelle le film flexible est soudé à la partie dure de la cassette autour de l'orifice d'aspiration.

27. Cassette médicale selon la revendication 26, dans laquelle le cordon de soudure annulaire autour de l'orifice d'aspiration comporte un profilage.

28. Cassette médicale selon la revendication 26, dans laquelle la zone du cordon de soudure est abaissée par rapport au niveau auquel la cassette est pressée.

29. Cassette médicale selon la revendication 24, dans laquelle un filtre hydrophobe est disposé sur l'orifice d'aspiration.

30. Procédé de couplage d'une cassette constituée d'une partie dure dotée de canaux de transfert de fluide, qui sont recouverts d'un film flexible, à la surface de couplage d'une machine de traitement destinée à traiter un fluide médical, comprenant les étapes consistant à
- coupler la cassette médicale à la surface de couplage de la machine de traitement,
- aspirer de l'air entre le film flexible et la surface de couplage de la machine de traitement pendant le procédé de couplage et/ou lorsque la cassette est couplée,
**caractérisé en ce que**
l'aspiration se fait le long d'un profilage de la surface du film flexible faisant face à la surface de couplage et/ou le long d'un profilage d'une surface d'un mat flexible faisant face au film flexible, qui est réalisé de manière étanche aux liquides et sans fente et par le biais duquel la cassette est couplée à la surface de couplage.

31. Procédé selon la revendication 30, comprenant une cassette médicale et/ou un dispositif destiné au traitement d'un fluide médical selon l'une des revendications précédentes.

32. Procédé selon la revendication 30 ou 31, destiné à vérifier l'étanchéité d'une cassette médicale constituée d'une partie dure dotée de canaux de transfert de fluide, qui sont recouverts d'un film flexible, en particulier avant le remplissage de la cassette médicale, comprenant les étapes consistant à
- coupler la cassette médicale à une surface de couplage d'une machine de traitement,
- aspirer de l'air entre le film flexible et la surface de couplage de la machine de traitement, en particulier aspirer au niveau de la surface pendant le procédé de couplage et/ou lorsque la cassette est couplée,
- vérifier l'étanchéité de la cassette médicale à l'aide de la dépression produite,
la vérification de l'étanchéité se faisant pendant et/ou après le couplage de la cassette.
